(19) Europäisches Patentamt
European Patent Office
Office européen des brevets

(11) EP 3 898 587 B1

(12) **EUROPEAN PATENT SPECIFICATION**

(45) Date of publication and mention of the grant of the patent:
**26.06.2024 Bulletin 2024/26**

(21) Application number: **19832675.3**

(22) Date of filing: **19.12.2019**

(51) International Patent Classification (IPC):
***C07D 209/52*** (2006.01) ***A61P 35/00*** (2006.01)
***A61P 37/00*** (2006.01) ***A61K 31/403*** (2006.01)

(52) Cooperative Patent Classification (CPC):
**C07D 209/52; A61P 35/00; A61P 37/00**

(86) International application number:
**PCT/EP2019/086482**

(87) International publication number:
**WO 2020/127846 (25.06.2020 Gazette 2020/26)**

(54) **CONDENSED PYRROLES AS NOVEL BROMODOMAIN INHIBITORS**

KONDENSIERTE PYRROLE ALS NEUARTIGE INHIBITOREN DER BROMODOMÄNE

PYRROLES CONDENSÉS UTILISÉS EN TANT QUE NOUVEAUX INHIBITEURS DE BROMODOMAINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018 EP 18215794**

(43) Date of publication of application:
**27.10.2021 Bulletin 2021/43**

(73) Proprietor: **Albert-Ludwigs-Universität Freiburg**
**79085 Freiburg (DE)**

(72) Inventors:
- **GÜNTHER, Stefan**
**79098 Freiburg (DE)**
- **WOHLWEND, Daniel**
**79133 Karlsruhe (DE)**
- **HÜGLE, Martin**
**79117 Freiburg (DE)**
- **LUCAS, Xavier**
**Dundee DDI 3BU (GB)**
- **REGENASS, Pierre-Marie**
**1906 Charrat (CH)**
- **PERVAIZ, Mehrosh**
**79114 Freiburg (DE)**
- **WARSTAT, Robin**
**79106 Freiburg (DE)**
- **BREIT, Bernhard**
**79194 Gundelfingen (DE)**
- **EINSLE, Olivier**
**79104 Freiburg (DE)**

(74) Representative: **Dr. Langfinger & Partner**
**In der Halde 24**
**67480 Edenkoben (DE)**

(56) References cited:
**WO-A1-2013/158952 WO-A1-2014/164771**

- **LISA A. HASVOLD ET AL: "Methylpyrrole inhibitors of BET bromodomains", BIOORGANIC & MEDICINAL CHEMISTRY LETTERS, vol. 27, no. 10, 1 May 2017 (2017-05-01), pages 2225-2233, XP055560115, AMSTERDAM, NL ISSN: 0960-894X, DOI: 10.1016/j.bmcl.2017.02.057**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 898 587 B1

## Description

The present invention relates to novel compounds which may be used as bromodomain inhibitors.

Bromodomains are epigenetic reader modules regulating gene transcription by recognizing acetyl-lysine modified histone tails.

The human genome encodes up to 61 different bromodomains (BRDs), present in transcriptional co-regulators and chromatin modifying enzymes including histone acetyl-transferases (HATs) and the Bromodomain extra-terminal domain (BET) family. They are epigenetic mark 'readers' that specifically recognize ε-*N*-acetylated lysine residues ($K_{ac}$).

Drug molecules that target epigenetic mechanisms of gene regulation are attractive as they offer the perspective of modifying processes responsible for dysfunctional malignant states rather than just treating the results thereof.

Bromodomains are small distinct domains within proteins that bind to acetylated lysine residues commonly but not exclusively in the context of histones.

BRDs fold into an evolutionary conserved four anti-parallel helix motif, linked by diverse loop regions of variable length (ZA and BC loops), which define the $K_{ac}$ binding site (Filippakopoulos et al. (2012), Cell 149(1), 214-231). In most BRDs, this site features an asparagine residue mainly responsible for substrate recognition (Owen DJ, et al. (2000), The structural basis for the recognition of acetylated histone H4 by the bromodomain of histone acetyltransferase gcn5p, The EMBO journal 19(22):6141-6149; Umehara T, et al. (2010) Structural basis for acetylated histone H4 recognition by the human BRD2 bromodomain, The Journal of biological chemistry 285(10):7610-7618).

The biological function of BRDs and their potential as therapeutic targets have been thoroughly described in literature (Muller S, Filippakopoulos P, & Knapp S (2011), Bromodomains as therapeutic targets, Expert reviews in molecular medicine 13:e29; Prinjha RK, Witherington J, & Lee K (2012), Place your BETs: the therapeutic potential of bromodomains, Trends in pharmacological sciences 33(3):146-153).

Research on the structural classification of bromodomains has revealed that the BET family of bromodomains has an overall highly targetable recognition site (Vidler LR, Brown N, Knapp S., Hoelder S (2012), Druggability analysis and structural classification of bromodomain acetyl-lysine binding sites, Journal of medicinal chemistry55(17):7346-7359*)*.

Members of the BET family, namely BRD2, BRD3, BRD4, and BRDT, modulate gene expression by recruiting transcriptional regulators to specific genomic locations. BRD4 and BRD2 have crucial roles in cell cycle control of mammalian cells. Along with BRD3, they are functionally linked to pathways important for cellular viability and cancer signalling and are co-regulators in obesity and inflammation. Specifically, BRD4 has been characterized as a key determinant in acute myeloid leukaemia, multiple myeloma, Burkitt's lymphoma, NUT midline carcinoma, colon cancer, and inflammatory disease. Because of its continued association with $K_{ac}$ in mitotic chromosomes, BRD4 has been postulated to be important for the maintenance of epigenetic memory.

Small molecules that inhibit bromodomains have potential as antiinflammatory, antiviral, and anticancer agents. Anticancer activity is mainly due to down-regulation of the key oncogene *c-MYC.* Recently, cytotoxicity in LAC cells has been related to suppression of the oncogenic transcription factor FOSL1 and its targets.

WO2014/164771 discloses compounds of formula

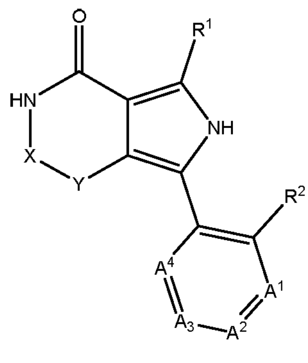

X-Y is -CR3=CH, -N=CR4, -CR5=N-, -CR6R7-CR8R9- wherein the left end of the moieties are attached to the NH group in the ring and $A^1$ to $A^4$ are individually $CR^x$ or one or two of $A^1$ to $A^4$ are N and the others are $CR^x$. The compounds are said to be bromodomain inhibitors.

Hasvold et al., Bioorganic & Medicinal Chemistry letters, Vol. 27, No. 10, 2225-2233 discloses a variety of methylpyrrole inhibitors of BET bromodomains comprising structural elements of formula

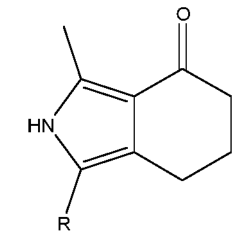

US 2015/111890 relates to isoindolone derivatives of the formula

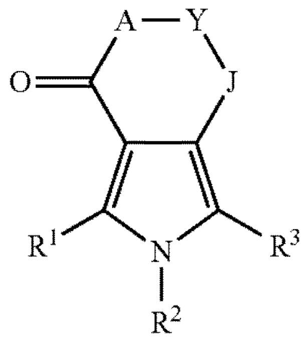

wherein A, Y and J each are a substituted or unsubstituted methylene group, $R^1$ is hydrogen or a $C_1$-$C_3$ alkyl group, $R^2$ is hydrogen or a $C_1$ to $C_3$ alkyl group and $R^3$ is an aryl or heteroaryl group. The compounds are said to show activity as bromodomain inhibitors.

US 2016/0200681 discloses on page 7 as intermediates in a reaction scheme compounds of formula

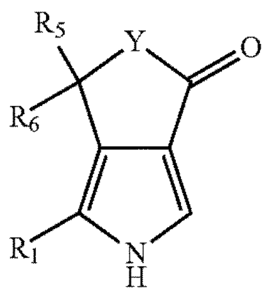

wherein Y is a $C_1$-$C_3$ alkylene group or-NH-, $R_1$ is i.a. a $C_1$-$C_4$ alkyl group and $R_5$ and $R_6$ are hydrogen or $C_1$-$C_4$ alkyl groups. The compounds are used for the treatment of peptic ulcer, gastritis or reflux esophagitis.

WO 2014/170350 relates to compounds of the formulae

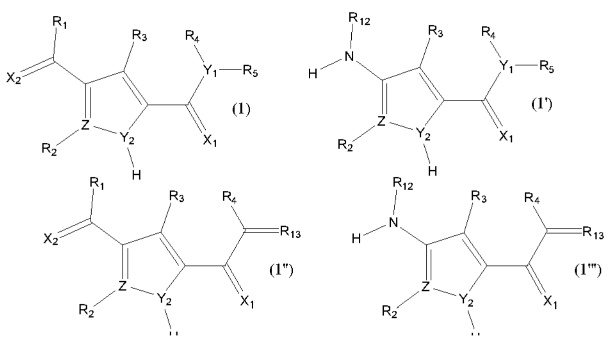

or a pharmaceutically acceptable salt thereof for use in the treatment of diseases or conditions for which a bromodomain inhibitor is indicated.

There is still a need for novel bromodomain inhibitors which may be used in the treatment of a variety of

diseases. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

Accordingly, it has been an object of the present invention to provide novel bromodomain inhibitors.

This object has been achieved with the compounds as defined in claim 1.

Preferred embodiments of the present invention are set forth in the dependent claims and in the detailed specification hereinafter.

Furthermore, another embodiment of the present invention relates to the use of the novel compounds of formula (I) as bromodomain inhibitors.

The novel compounds in accordance with the present invention are represented by formula (1) or (2)

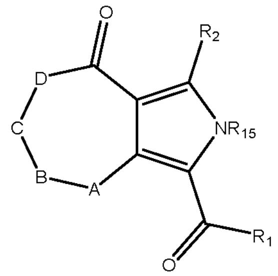

(1)

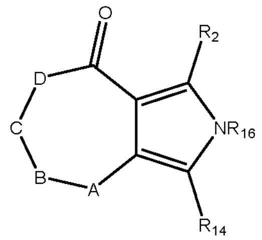

(2)

wherein

A, B, C and D, which may be the same or different, independent of one another represent $-C(R_7R_8)-$, $-C(R_9R_{10})-C(R_{11}R_{12})-$ , $-N(R_{13})-$, -O- or -S-with the proviso that the total number of ring atoms of the ring comprising ring members A, B, C and D is 7 to 9,

$R_1$ is selected from the group consisting of OH, $OR_3$, $NH_2$, $NHR_4$ and $NR_5R_6$

$R_2$ is a substituted or unsubstituted alkyl group with 1 to 8 carbon atoms or a substituted or unsubstituted alkoxy group with 1 to 8 carbon atoms in which one or more of the hydrogen atoms may be optionally replaced by halogen,

$R_3$ and $R_4$, independent of each other, represent an alkyl group with 1 to 6 carbon atoms or a substituted or unsubstituted alkylaryl, aryl or heteroaryl group,

$R_5$ and $R_6$, independent of each other, represent an alkyl group with 1 to 6 carbon atoms or a substituted or unsubstituted aryl or heteroaryl group or form together with the nitrogen atom to which they are attached, a six- or seven membered, substituted or unsubstituted heterocyclic ring,

$R_7$ to $R_{13}$, independent of each other, represent hydrogen, hydroxyl, substituted or unsubstituted alkyl groups with 1 to 4 carbon atoms, substituted or unsubstituted alkoxy groups with 1 to 4 carbon atoms and substituted or unsubstituted hydroxyalkyl groups with 1 to 4 carbon atoms or wherein two of $R_7$ to $R_{13}$ located at adjacent atoms form together a chemical bond or a ring,

$R_{14}$ represents a substituted or unsubstituted aryl or heteroaryl group, and

$R_{15}$ and $R_{16}$, independent of each other, are hydrogen or a $C_1$-$C_4$-alkyl group, preferably $R_{15}$ and $R_{16}$ represent hydrogen.

The term substituted or unsubstituted divalent alkylene group with 2 to 4 carbon atoms, when used herein, represents a group $-(CR'R")_n$ - wherein n is an integer from 2 to 4 and R' and R", independent of each other represent hydrogen, a $C_1$ to $C_4$ alkyl group or a $C_1$-$C_4$ alkoxy group.

The term $C_1$-$C_n$ alkyl group, when used herein, represents a linear or branched hydrocarbon group with 1 to n carbon atoms. Examples are methyl, ethyl, *i*-propyl, *n*-propyl, *i*-butyl, *n*-butyl and *t*-butyl, to name only a few representatives.

One or more hydrogen atoms in the alkyl groups can be replaced by substituents consisting of halogen, alkyl,

alkoxy, amino, cyano, alkenyl, alkynyl, arylalkyl, aryl, and heteroaryl groups.

The term $C_1$ to $C_8$ alkoxy group, when used herein, represents a derivative of an alkyl group comprising at least one O-C bond. Representative examples are $OCH_3$, $OC_2H_5$, $OC_3H_7$ and $OC_4H_9$. One or more hydrogen atoms attached to the carbon atoms in the alkoxy groups can be replaced by substituents consisting of halogen, alkyl, alkoxy, amino, cyano, alkenyl, alkynyl, arylalkyl, aryl, and heteroaryl groups.

The term $C_1$-$C_8$ hydroxyalkyl group, when used herein, represents a linear or branched $C_1$-$C_8$-alkyl group in which one or more of the hydrogen atoms are replaced by a hydroxy group. One or more of the remaining hydrogen atoms attached to the carbon atoms in the hydroxyalkyl groups can be replaced by substituents consisting of halogen, alkyl, alkoxy, amino, cyano, alkenyl, alkynyl, arylalkyl, aryl, and heteroaryl groups.

In accordance with a preferred embodiment, at least one, preferably at least two, more preferably at least three of groups A, B, C and D represent a group -($CR_7R_8$)- or a methylene group (-$CH_2$-). Particularly preferred A, B, C and D are all methylene groups.

In accordance with another preferred embodiment $R_2$ is an alkyl group with 1 to 4 carbon atoms, preferably a methyl group, an ethyl group, a propyl group or a butyl group. In some cases a methyl group has been found to be advantageous.

$R_1$ in the compounds of formula (1) preferably is a group $OR_3$, $NHR_4$ or a substituted aryl group. The aryl group is preferably a phenyl group, which may be unsubstituted or may carry one or more substituents. Preferred substituents of the aryl, respectively phenyl groups are alkoxy groups, groups -S(=O)$_2$-R with R being preferably an amino group, a heterocyclic ring with 4 to 7 carbon atoms or a carboxamido group.

If $R_1$ is $OR_3$ or $NHR_4$, $R_3$ and $R_4$ preferably represent an alkyl group with 1 to 4 carbon atoms.

A preferred group of substituents $R_4$ in compounds of formula (1) is represented by formula (3)

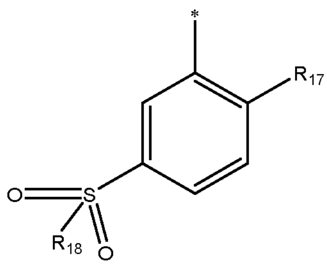

(3)

wherein * represents the site of attachment to the nitrogen atom, $R_{17}$ is hydroxyl or an alkoxy group with 1 to 4 carbon atoms and $R_{18}$ is -$NR_{19}R_{20}$ wherein $R_{19}$ and $R_{20}$, independent of each other, represent an alkyl group with 1 to 4 carbon atoms, a substituted or unsubstituted aryl ring or together with the nitrogen atom to which they are attached form a substituted or unsubstituted heterocyclic ring with 4 to 7 ring atoms.

In accordance with still another preferred embodiment, $R_{14}$ in the compounds of formula (2) is a substituted or unsubstituted phenyl, oxadiazole or indole group. Preferred substituents at these rings are amino, hydroxyl, halogen, carboxamide and alkyl groups.

Further preferred compounds of formula (1) in accordance with the present invention are represented by formulae (4) to (7)

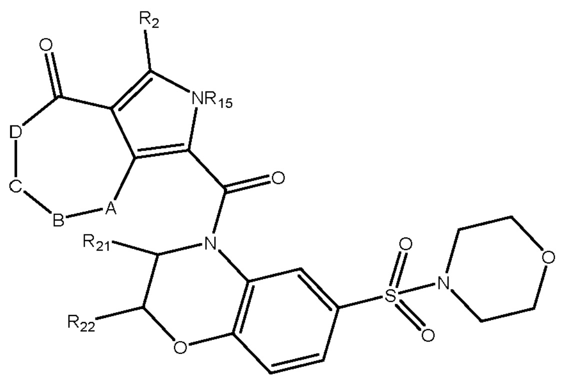

(4)

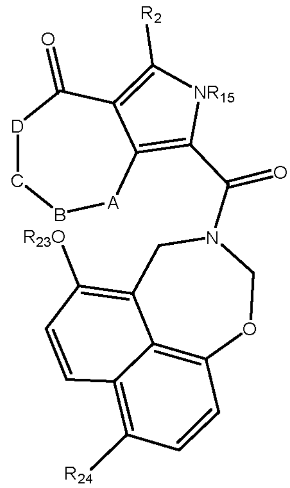

(5)

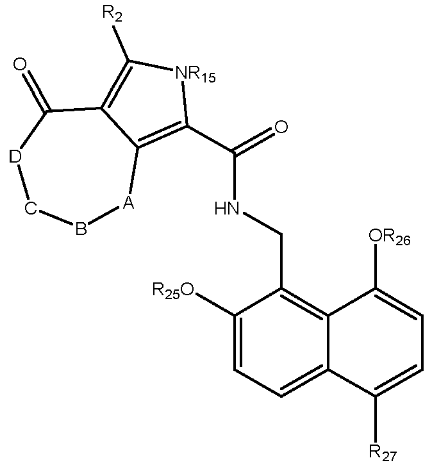

(6)

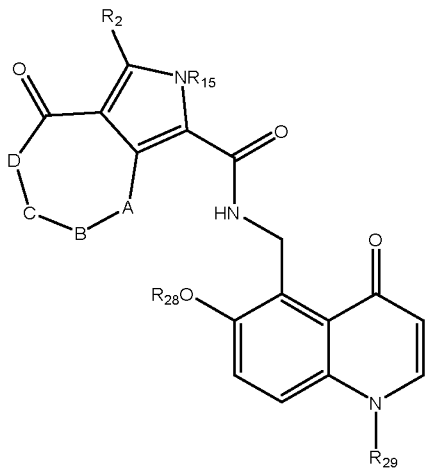

(7)

wherein A, B, C, D, $R_2$ and $R_{15}$ are as defined in claim 1,

$R_{21}$ and $R_{22}$, independent of each other, are hydrogen, $C_1$-$C_4$ alkyl or form together a chemical bond,
$R_{23}$, $R_{25}$ and $R_{23}$, independent of each other, are hydrogen or $C_1$ to $C_4$ alkyl,
$R_{24}$, $R_{27}$ and $R_{29}$, independent of each other, are $C_1$-$C_4$ alkyl or $C_2$-$C_4$-carboxyl, and
$R_{26}$ is hydrogen or $C_1$ to $C_4$ alkyl.

The preferred embodiments for the substituents in formula (4) to (7) are as defined for the preferred embodiments described hereinbefore.

Preferred examples of compounds of formula (2) in accordance with the present invention are represented by formula (2')

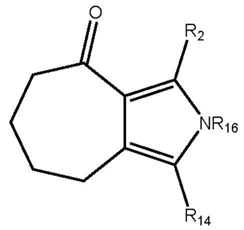

(2')

wherein $R_2$, $R_4$ and $R_{16}$ are as defined in claim 1.

In the compounds of formula (2) and (2'), $R_2$ is preferably an alkyl group with 1 to 4 carbon atoms. $R_{14}$ is preferably a substituted or unsubstituted phenyl or a substituted oxadiazole group. $R_{16}$ is preferably hydrogen. Preferred substituents of the phenyl or oxadiazole group are OH, $NH_2$, halogen, carboxamide or alkyl. The phenyl or oxadiazole groups in the preferred compounds of formula (2') may carry one or more than one substituent, with a combination of halogen and amino groups being particularly preferred (such as e.g. in compounds (21), (26) and (27) below).

The following compounds (8) to (27) represent particularly preferred embodiments of the compounds of the present invention, wherein formulae (8) to (18) are representative of compounds of formula (1) and formulae (19) to (27) are representative of compounds of formula (2):

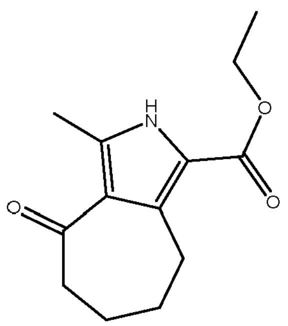

(8)

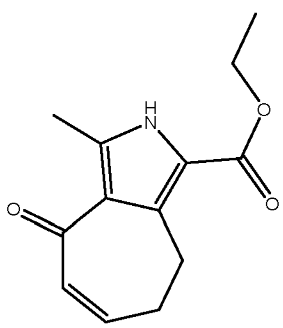

(9)

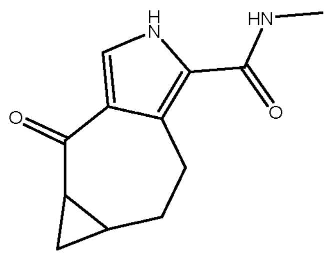

(10)

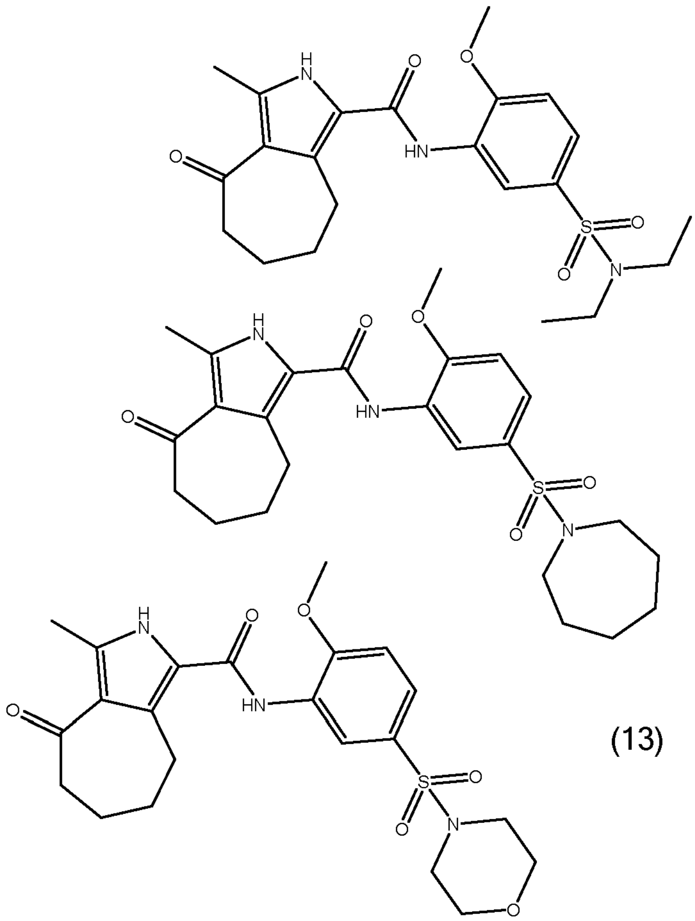

(11)

(12)

(13)

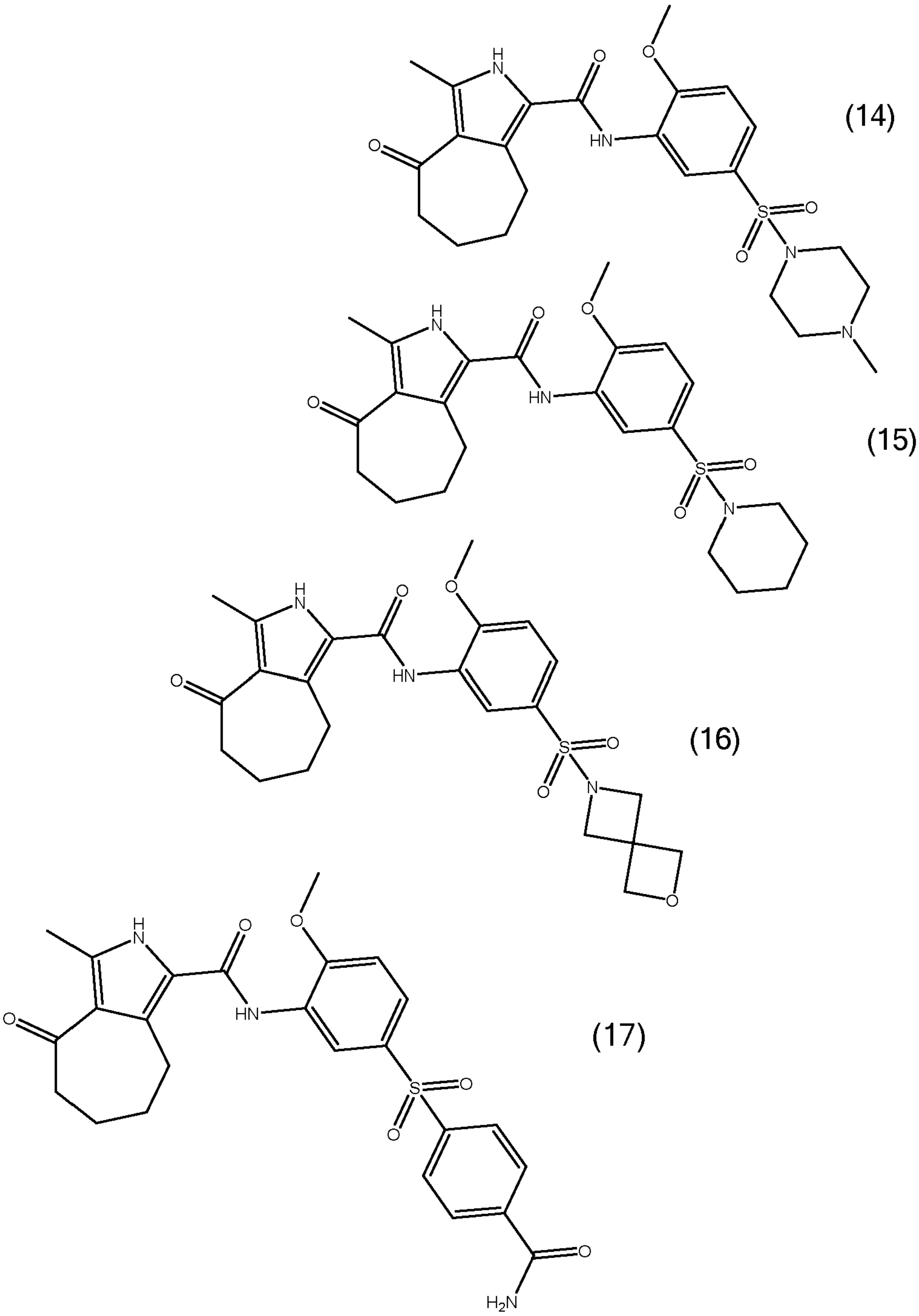

(14)

(15)

(16)

(17)

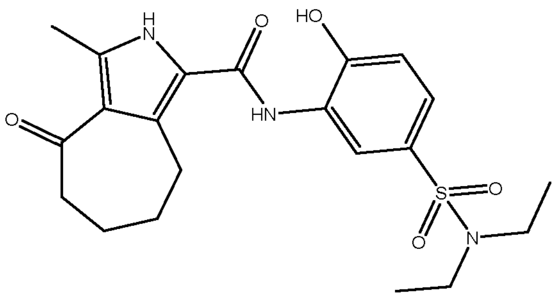

(18)

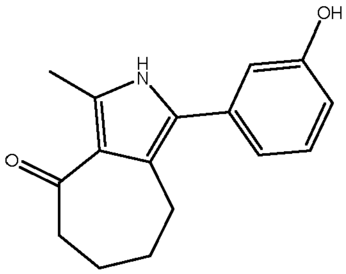

(19)

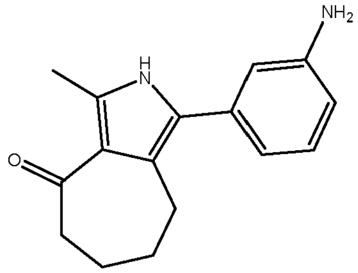

(20)

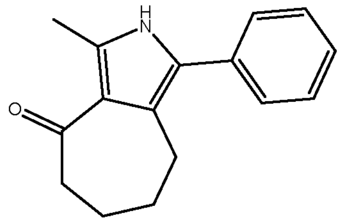

(21)

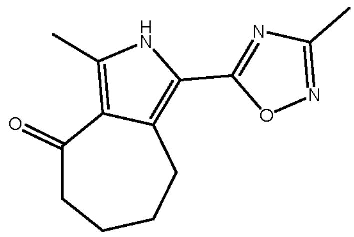

22

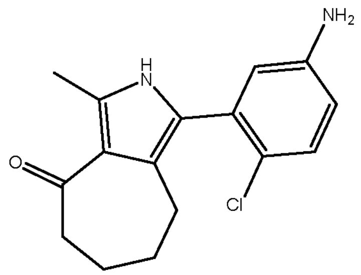

(23)

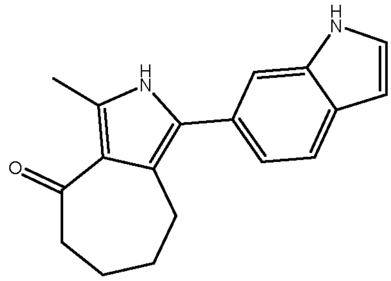

(24)

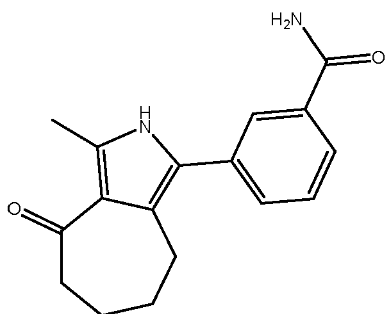

(25)

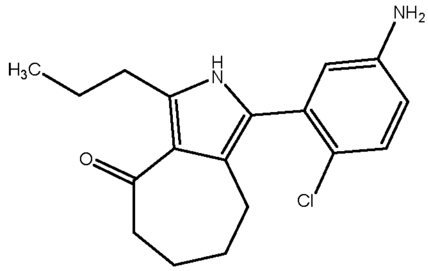

(26 )

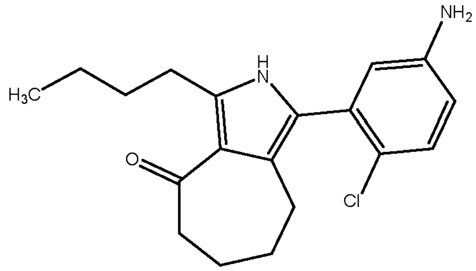

(27)

Another group of preferred compounds of formula (1) are represented by formula (1')

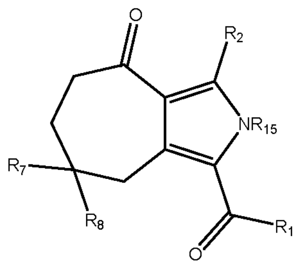

(1')

wherein $R_1$, $R_2$, $R_7$, $R_8$ and $R_{15}$ are as defined in claim 1.

In accordance with another preferred embodiment, $R_2$ is a substituted or unsubstituted alkyl group with 1 to 4 carbon atoms.

In accordance with still another preferred embodiment, $R_1$ is a group $NHR_6$ wherein $R_6$ is as defined in claim 1.

In accordance with another preferred embodiment, $R_{15}$ is hydrogen.

A particularly preferred compound of formula (1) in accordance with the present invention is represented by formula (1")

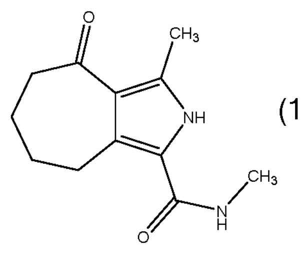

(1")

In accordance with the present invention the compounds of formula (1) or (2) may be used in the form of their pharmaceutically acceptable salts. The skilled person is aware of pharmaceutically acceptable salts of compounds of formula (1) or (2) and will select the appropriate salt based on his professional experience and knowledge. Suitable pharmaceutically acceptable salts can include acid or base addition salts. For a review on suitable salts see Berge et al. (1977) J. Pharm.Sci.66:1-19. Typically, pharmaceutical acceptable salts may be readily prepared by using a desired acid or base as appropriate. The resultant salt may precipitate from solution and be collected by filtration or may be recovered by evaporation of the solvent.

A pharmaceutically acceptable base addition salt can be formed by reaction of a compound of formula (1) or

(2) with a suitable inorganic or organic base, optionally in a suitable solvent, to give the base addition salt which is usually isolated, for example by crystallization and filtration. Pharmaceutically acceptable base salts include ammonium salts, alkali metal salts, alkaline earth metal salts and salts with organic bases, in particular salts with primary, secondary and tertiary amines, e.g. isopropyl amine, diethylamine, ethanolamine, trimethylamine and dicyclohexylamine.

The pharmaceutically acceptable acid addition salt can be formed by reaction of a compound of formula (1) or (2) with a suitable inorganic or organic acid, in a suitable solvent such as an organic solvent, to give the product which is usually isolated, for example, by crystallization and filtration. Pharmaceutically acceptable acid addition salts of a compound of formula (I) can comprise or can be, for example, a nitrate, sulphate, hydrobromide, hydrochloride, phosphate, maleate, acetate, propionate, fumarate, citrate, tartrate, salicylate, aspartate, succinate, benzoate, p-toluenesulphonate, methanesulphonate, naphthalenesulphonate, ethanesulphonate or hexanoate salt.

In accordance with the present invention the pharmaceutically acceptable salts include all possible stoichiometric and non-stoichiometric forms of the salts of the compounds of formula (1) or (2).

The compounds of formula (1) or (2) form complexes with solvents in which they are reacted or from which they are precipitated or crystallized, which complexes are generally referred to as solvates. In accordance with the present invention all possible stoichiometric and non-stoichiometric forms of the solvates of the compounds of formula (1) or (2) are included.

The present invention also encompasses all prodrugs of the compounds of formula (1) or (2) and pharmaceutically acceptable salts thereof, which upon administration to the recipient are capable of providing (directly or indirectly) a compound of formula (1) or (2) or a pharmaceutically acceptable salt thereof or an active metabolite or acceptable salt thereof. In this regard, reference is made to Burger's Medicinal Chemistry and drug discovery, 5th Ed., Vol. 1, Principles and Practice, to which reference is made here for further details.

The compounds of formula (1) or (2) may be in crystalline or amorphous form. Furthermore, some of the crystalline forms may exist in different morphological conformations, which are included within the scope of the present invention. Different morphological forms (polymorphic forms) of the compounds of formula (1) or (2) can be characterized and different analytical techniques are known to the person skilled in the art so that no further details are necessary here.

The compounds of formula (1) or (2) may also exist in one of several tautomeric forms and in accordance with the present invention individual tautomers or mixtures thereof are encompassed.

The compounds of formula (1) or (2) or their pharmaceutically acceptable salts in accordance with another embodiment of the present invention are used in the treatment of diseases or conditions for which a bromodomain inhibitor is indicated.

Bromodomain inhibitors are believed to be useful in the treatment of a variety of diseases or conditions related to systemic or tissue inflammation, inflammatory responses to infection or hypoxia, cellular activation and proliferation, lipid metabolism, fibrosis and in the prevention and treatment of viral infections.

Bromodomain inhibitors may thus be useful in the treatment of a great variety of chronic autoimmune and inflammatory conditions such as rheumatoid arthritis, osteoarthritis, acute gout, psoriasis, systemic lupus erythematosus, multiple sclerosis, inflammatory bowel disease, asthma, chronic obstructive airways disease, pneumonitis, myocarditis, pericarditis, myositys, eczema, dermatitis, alopecia, vitiligo, bullous skin diseases, nephritis, vasculitis, atherosclerosis, Alzheimer's disease, depression, retinitis, uveitis, scleitis, hepatitis, pancreatitis, primary biliary cirrhosis, sclerosis, cholangitis, Addison's disease, hypophysitis, thyroiditis, type I diabetes and acute rejection of transplanted organs.

The compounds in accordance with formula (1) or (2) may also be useful in the prevention or treatment of diseases or conditions which involve inflammatory responses to infections with bacteria, viruses, fungi, parasites or the like, such as sepsis, sepsis syndrome, septic shock, endotoxemia, systemic inflammatory response syndrome (SIRS), multi-organ dysfunction syndrome, toxic shock syndrome, acute lung injury, adult respiratory distress syndrome, acute renal failure, fulminant hepatitis, burns, acute pancreatitis, post-surgical syndromes, sarcoidosis, Herxheimer reactions, encephalitis, myelitis, meningitis, malaria, and SIRS associated with viral infections such as influenza, herpes zoster, herpes simplex, and coronavirus.

The compounds of formula (1) or (2) may also be used in the treatment of disorders of the lipid metabolism involving the regulation of APO-A1, such as hypercholesterolemia, atherosclerosis, and Alzheimer's disease.

Furthermore, the compounds of formula (1) or (2) may be used in the prevention or treatment of conditions associated with ischemia-reperfusion injury such as myocardial infarction, cerebrovascular ischemia (stroke), acute coronary syndromes, renal reperfusion injury, organ transplantation, coronary artery bypass grafting, cardio-pulmonary bypass procedures and pulmonary, renal, hepatic, gastro-intestinal or peripheral limb embolism.

Bromodomain inhibitors may further be useful in the treatment of fibrotic conditions such as idiopathic pulmonary fibrosis, renal fibrosis, postoperative stricture, keloid formation, scleroderma and cardiac fibrosis.

A further indication for bromodomain inhibitors is the prevention and treatment of viral infections such as herpes virus, human papilloma virus, adenovirus, poxvirus and other DNA viruses.

The compounds of formula (1) or (2) are useful in the treatment of cancer, including but not limited to types of cancer included in the NCI60 panel, such as bladder cancer, brain cancer, breast cancer, cervical carcinoma, colorectal

cancer, oesophageal cancer, gastric cancer, head and neck cancer, leukaemia, lymphoma, NSCLC cancer (non-small cell lung carcinoma), ovarian cancer, pancreatic cancer, sarcoma, SCLC cancer (small cell lung cancer), melanoma, renal cancer, prostate cancer and hepatocellular carcinoma. including haematological (such as leukaemia), epithelial including lung, breast and colon carcinomas, midline carcinomas, mesenchyme, hepatic, renal and neurological tumours.

Preferred uses of the compounds of formula (1) or (2) are the treatment of chronic autoimmune or inflammatory conditions, cancer or viral diseases.

The compounds of formula (1) or (2) can also be preferably used for the inhibition of proliferation of leukaemia cells or for the treatment of a disease or condition which is obesity or a kidney malfunction.

Furthermore, the compounds of formula (1) or (2) could also be effective as male contraceptives.

The compounds of formula (1) or (2) can also be preferably used for the treatment of diseases caused by parasites such as malaria or leishmaniasis.

Compounds of formula (1) or (2) can also be used in the treatment of diseases or conditions related to bromodomains CREBBP and EP300. By way of example, acute myeloid leukaemia, acute lymphoblastic leukaemia, non-Hodgkin lymphoma, prostate cancer and spinal and bulbar muscular atrophy may be mentioned in this regard.

In the course of the studies leading to the present invention it has been recognized that the compounds of formula (1) or (2) in particular are effective in inhibiting the bromodomains of the BET family, including BRD2-BD1, BRD2-BD2, BRD3-BD1, BRD3-BD2, BRD4-BD1, BRD4-BD2 BRDT-BD1, BRDT-BD2 as well as the bromodomains , BRD7, BRD9, BRPF1, BAZ2A, BAZ2B, BRD1, BRD8-BD1, CECR2, FALZ, PBRM1-BD2, BPRM1-BD5, TAF1-BD2, TAF1L-BD2, TRIM24, TRIM33, CREBBP and EP300, with a particularly good effect in the inhibition of the mentioned bromodomains of the BET family. In some cases, particularly good results were obtained in the inhibition of bromodomains BRD7 and BRD9.

In accordance with the present invention, the compounds of formula (1) or (2) as well as their pharmaceutically acceptable salts may be administered as such or may be presented as active ingredients in a pharmaceutical composition.

Accordingly, a further embodiment of the present invention relates to a pharmaceutical composition comprising at least one compound of formula (1) or (2) or a pharmaceutically acceptable salt thereof and one or more pharmaceutically acceptable carriers, diluents or excipients.

The carriers, diluents or excipients used in pharmaceutical compositions must be acceptable in the sense of being compatible with the other ingredients of the composition and not detrimental to the recipient.

Such pharmaceutical compositions may be obtained by mixing a compound of formula (1) or (2) or a pharmaceutically acceptable salt thereof, with one or more pharmaceutically acceptable carriers, diluents or excipients.

Since the compounds and pharmaceutically acceptable salts thereof are intended for use in pharmaceutical compositions it is apparent that they are preferably provided in substantially pure form, i.e. with a degree of impurities as low as possible. Preferably the purity is at least 60%, more preferably at least 75% and most preferably at least 98% (in each case per cent by weight).

Pharmaceutical compositions may be presented in unit dose forms containing a predetermined amount of active ingredient per unit dose. Preferred unit dosage compositions are those containing a daily dose or sub-dose, or an appropriate fraction thereof, of an active ingredient. Such unit doses may therefore be administered more than once a day. Preferred unit dosage compositions are those containing a daily dose or sub-dose as recited above.

The pharmaceutical compositions may be adapted for administration by any appropriate route, for example by the oral, rectal, inhalation, intranasal, topical, vaginal or parenteral route.

The skilled person will select the appropriate administration route based on the conditions of the specific application. The manufacture of the appropriate administration forms is known to the skilled person so that no further details are necessary here.

When desired or necessary, suitable binders, glidants, lubricants, sweetening agents, flavours, disintegrating agents and colouring agents can be included in the pharmaceutical composition. The skilled person will select the appropriate additives based on his professional experience and in adaptation to the specific application.

The pharmaceutical compositions in accordance with the present invention may comprise more than one active ingredient, e.g. more than one compound of formula (1) or (2) or a pharmaceutically acceptable salt thereof or a combination of a compound of formula (1) or (2) with other active ingredients or their pharmaceutically acceptable salts.

In particular combinations of bromodomain inhibitors of formula (1) or (2) and histone deacetylase inhibitors (HDAC inhibitors) provide a very interesting property spectrum and combination of efficiencies.

Various HDAC inhibitors useful to be combined with the compounds of formula (1) or (2) have been investigated and described in the literature and are known to the skilled person.

Vorinostat and Romidepsin have been approved by the US FDA for cutaneous T-cell lymphoma.

Panobinostat, valproic acid and belinostat are in phase III clinical trials for various types of cancer.

Mocetinostat, Abexinostat, Entinostat, Resminostat, Givinostat, Qusinostat and SB 939 are in phase II clinical trials.

CUDC-101, AR-42, ACY-1215, Kevetrin, Trichostatin A are in early stage development as HDAC inhibitors.

All the aforementioned HDAC inhibitors can principally be combined with the compounds of formula (1) or (2) in accordance with the present invention to obtain pharmaceutical compositions.

The compounds of formula (1) or (2) for use in accordance with the present invention present a novel class of interesting active molecules in the epigenetic field and in particular may be considered as a representative of a new class of bromodomain inhibitors of the BET family.

The skilled person is aware of suitable methods for the synthesis of compounds of formula (1) or (2) so that there is no further description necessary here.

The compounds of formula (1) or (2) or a pharmaceutically acceptable salt thereof may be used in the manufacture of a medicament for the treatment of diseases or conditions for which a bromodomain inhibitor is indicated. In other embodiments, there is provided the use of a compound of formula (1) or (2) or a pharmaceutically acceptable salt thereof in the manufacture of a medicament for the treatment of a chronic autoimmune and/or inflammatory condition or in the treatment of cancer.

In a further embodiment there is provided a method for treatment of a disease or condition, for which a bromodomain inhibitor is indicated, in a subject in need thereof which comprises administering a therapeutically effective amount of a compound of formula (1) or (2) or a pharmaceutically acceptable salt thereof.

In one embodiment the subject in need of treatment is a mammal.

The term effective amount means an amount of a drug or pharmaceutical agent that will elicit the biological or medical response of a tissue, system, animal or human that is being sought, for instance by a researcher or a clinician. Furthermore, the term therapeutically effective amount means any amount which, as compared to a corresponding subject which has not received such amount, results in improved treatment, healing, prevention or amelioration of a disease, disorder, or side effect, or a decrease in the rate of advancement of a disease or disorder. The term also includes within its scope amounts effective to enhance normal physiological function.

The following examples represent embodiments of the present invention. The skilled person will easily recognize that he can modify the structure of the compounds in the working examples in an appropriate manner.

## Examples

### General Procedures

Reagents and solvents were obtained from commercial sources and used without any further purification.

Column chromatography was accomplished using MACHEREY-NAGEL silica gel 60® (230-400 mesh). Thin layer chromatography was performed on aluminum plates pre-coated with silica gel (MERCK, $60F_{254}$), which were visualized by UV fluorescence ($\lambda_{max}$ = 254 nm) and/or by staining with ninhydrin in ethanol (EtOH).

### $^1H$ and $^{13}C$ NMR Spectroscopy

NMR spectra were acquired on a BRUKER Avance 400 spectrometer (400 MHz and 100.6 MHz for $^1H$ and $^{13}C$ respectively) or a Bruker 500 DRX NMR spectrometer with TBI probe head (499.6 MHz and 125.6 MHz for $^1H$ and $^{13}C$ respectively) at a temperature of 303 K unless specified. Chemical shifts are reported in parts per million (ppm) relative to residual solvent. Data for $^1H$ NMR are described as following: chemical shift ($\delta$ in ppm), multiplicity (s, singlet; d, doublet; t, triplet; q, quartet; m, multiplet; br, broad signal), coupling constant (Hz), integration. Data for $^{13}C$ NMR are described in terms of chemical shift ($\delta$ in ppm).

High Resolution Mass Spectrometry (HR-MS) data were obtained on a THERMO SCIENTIFIC Advantage and a THERMO SCIENTIFIC instrument (Atmospheric Pressure Chemical Ionization (APCI)/methanol (MeOH): spray voltage 4-5 kV, ion transfer tube: 250-300 °C, vaporizer: 300-400 °C).

### Example 1 Synthesis of 3-methyl-4-oxo-2,4,5,6,7,8-hexahydrocyclohepta[c]pyrrole-1-carboxylic acid

General Synthesis Scheme

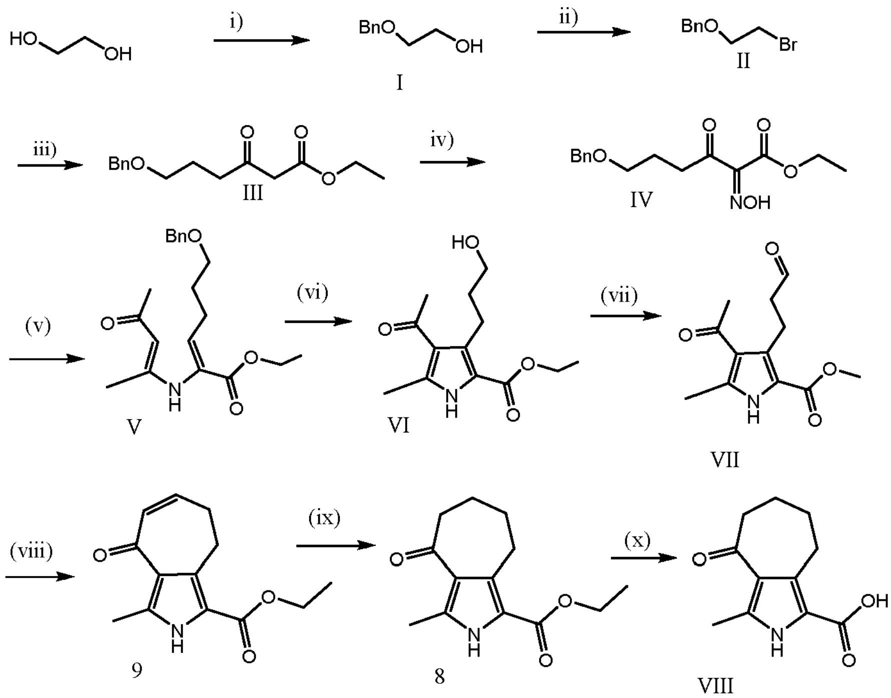


I II III IV V VI VII 9 8 VIII

Reaction conditions: i) Benzyl bromide (BnBr), sodium hydride (NaH), THF, room temperature (room temperature, hereinafter referred to as rt), 93% ; ii) N-Bromosuccinimide (NBS), triphenylphosphine ($PPh_3$), dichloromethane ($CH_2Cl_2$), -78 °C to rt, 89% ; iii) Ethyl acetoacetate, NaH, n-butyllithium (BuLi), -20 °C to rt, 76% ; iv) sodium nitrite ($NaNO_2$), acetic acid (AcOH)/$H_2O$, 0 °C to rt, 96% ; v) Pentane-2,4-dione, Zn, AcOH, sodium acetate (AcONa), 90 °C, 63% ; vi) $H_2$, palladium on charcoal (Pd/C), ethyl acetate (EtOAc)/(MeOH), rt, 67% ; vii) $(COCl)_2$, triethylamine (NEts), DMSO, $NEt_3$, $CH_2Cl_2$, -78 °C to rt, 100% ; viii) p-toluene sulfonic acid (PTSA).$H_2O$, toluene (PhMe), 110 °C, 56% ; ix) $H_2$, Pd/C, EtOAc/MeOH, rt, 98% ; x) NaOH, EtOH/$H_2O$, reflux, then HCl, 0 °C, 71%

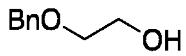 Compound I

**2-(benzyloxy)ethan-1-ol** (Compound **I**). To a suspension of NaH (10.2 g, 0.25 mmol, 1.05 equiv) in dry THF (152 mL) ethylene glycol (250 g, 2.42 mol, 10 equiv) was added dropwise at 0 C. The reaction mixture was first stirred at rt for 30 min and afterwards benzylbromide (48 mL, 0.24 mol, 1.0 equiv) was added at 0 C. The mixture was stirred for 12 h at rt and afterwards quenched with saturated ammonium chloride ($NH_4Cl$) solution. The aqueous phase was extracted three times with diethyl ether ($Et_2O$). The combined organic layers were washed with $H_2O$, brine, dried over sodium sulfate ($Na_2SO_4$) and the solvent was evaporated under reduced pressure. The residue was purified by column chromatography (20-100% EtOAc in petroleum ether (hereinafter referred to as PE) to afford the desired compound as a light yellow oil (56.7 g, 373 mmol, **93**%). **$^1$H NMR** (400 MHz, $CDCl_3$) δ .39 - 7.27 (m, 5H), 4.56 (s, 2H), 3.79 - 3.69 (m, 2H), 3.61 - 3.54 (m, 2H), 2.61 - 2.50 (m, 1H); **$^{13}$C NMR** (101 MHz, $CDCl_3$) δ 138.0, 128.4, 127.8, 127.7, 73.2, 71.5, 61.8; **$R_f$:** 0.39 (40% AcOEt in PE).

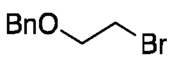 Compound II

**((2-bromoethoxy)methyl)benzene** (Compound **II**). To a solution of NBS (17.7 g, 99.3 mol, 1 equiv.) in $CH_2Cl_2$ (200 mL) was added dropwise a solution of $PPh_3$ (26.1 g, 99.3 mol, 1 equiv.) in $CH_2Cl_2$ (140 mL) at -78 °C. The solution was stirred for 1 h, then a solution of 2-(benzyloxy)ethan-1-ol (14.4 g, 94.6 mmol, 1 equiv.) in $CH_2Cl_2$ (100 mL) was added dropwise. The solution was allowed to return to rt and after one hour, the reaction was quenched using MeOH (10 mL) and PhMe (150 mL). The solution was evaporated, water was added and the aqeous phase was extracted three times with EtOAc. The combined organics were washed with brine, dried over $Na_2SO_4$ and evaporated. The residue was purified on silica gel column eluting with 10% EtOAc in cyclohexane to afford the desired product (18.1 g, 84.1 mol,

**89**%) as a light yellow oil.

**$^1$H NMR** (400 MHz, $CDCl_3$) δ 7.41 - 7.28 (m, 5H), 4.60 (s, 2H), 3.80 (t, *J* = 6.2 Hz, 2H), 3.50 (t, *J* = 6.2 Hz, 2H); **$^{13}$C NMR** (101 MHz, $CDCl_3$) δ 137.8, 128.5, 127.9, 127.8, 73.2, 70.0, 30.5; **$R_f$:** 0.85 (20% AcOEt in CyH)

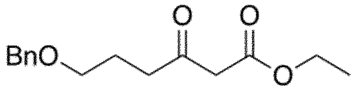

Compound III

**Ethyl 6-(benzyloxy)-3-oxohexanoate** (Compound **III**). Ethyl acetoacetate (32 mL, 252 mmol, 1.0 equiv) was added dropwise to a suspension of NaH (5.55 g, 278 mmol, 1.1 equiv, 60% w/w) in dry THF (500 mL) at -20 C. The reaction mixture was stirred for 1 h, then BuLi in hexane (2.5 M, 112 mL, 278 mmol, 1.1 equiv) was added dropwise. After 1h, ((2-bromoethoxy)methyl)benzene (65.1 g, 302 mmol, 1.0 equiv) was added and the reaction mixture was allowed to return to rt and stirred overnight. A saturated $NH_4Cl$ solution was added and the organic phase was evaporated. The aqueous phase was extracted three times with EtOAc. The combined organic layers were washed with brine, dried over $Na_2SO_4$ and evaporated. The residue was purified by column chromatography eluting with 10-100% EtOAc in PE to afford the desired compound as an orange liquid (50.5 g, 191 mmol, **76**%). **$^1$H NMR** (300 MHz, $CDCl_3$) δ 7.41 - 7.28 (m, 5H), 4.50 (s, 2H), 4.20 (q, *J* = 7.1 Hz, 2H), 3.51 (t, *J* = 6.1 Hz, 2H), 3.45 (s, 2H), 2.68 (t, *J* = 7.1 Hz, 2H), 1.99 - 1.89 (m, 2H), 1.29 (t, *J* = 7.1 Hz, 3H); **$^{13}$C NMR** (101 MHz, $CDCl_3$) δ 202.6, 167.3, 138.5, 128.5, 127.7, 127.7, 73.0, 69.1, 61.4, 49.5, 39.8, 23.8, 14.2; **HRMS** (ESI) : calcd. for $C_{15}H_{20}O_4Na$ $[M+Na]^+$: 287.1254, found : 287.1254; **$R_f$:** 0.30 (20% AcOEt in cyclohexane (CyH))

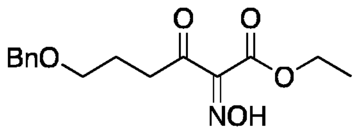

Compound IV

**Ethyl (Z)-6-(benzyloxy)-2-(hydroxyimino)-3-oxohexanoate** (Compound IV). To a solution of ethyl-6-(benzyloxy)-3-oxohexanoate (63.0 g, 238 mmol, 1.0 equiv) in AcOH (240 mL) at 0 C, was added dropwise a solution of $NaNO_2$ (32.9 g, 477 mmol, 2.0 equiv) in $H_2O$ (125 mL). The mixture was then allowed to return to rt, and stirred overnight. Water was added and the aqueous phase was extracted four times with ethyl acetate (EA). The combined organic layers were washed three times with saturated $Na_2CO_3$ solution, brine and dried over $Na_2SO_4$ to afford the desired compound as a yellow oil (67.0 g, 229 mmol, 96%). $^1$H NMR (400 MHz, $CDCl_3$) δ 10.51 (s, 1H), 7.42 - 7.26 (m, 5H), 4.53 (s, 2H), 4.35 (q, *J* = 7.1 Hz, 2H), 3.55 (td, *J* = 6.1, 1.7 Hz, 2H), 2.87 (dt, *J* = 35.4, 7.2 Hz, 2H), 2.09 - 1.78 (m, 2H), 1.33 (t, *J* = 7.1 Hz, 3H); $^{13}$C NMR (101 MHz, $CDCl_3$) δ 195.6, 161.9, 150.6, 137.5, 128.5, 127.9, 127.9, 73.0, 69.2, 62.2, 34.4, 23.6, 14.0; HRMS (ESI) : calcd. for $C_{15}H_{19}NO_5Na$ $[M+Na]^+$ : 316.1155, found : 316.1155; $R_f$: 0.63 (33% AcOEt in CyH)

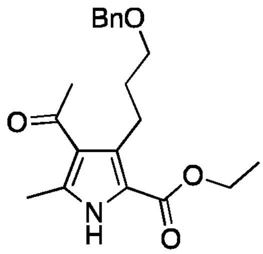

Compound V

**Ethyl 4-acetyl-3-(3-(benzyloxy)propyl)-5-methyl-1*H*-pyrrole-2-carboxylate (Compound V).** To a solution of ethyl (Z)-6-(benzyloxy)-2-(hydroxyimino)-3-oxohexanoate (28.1 g, 95.9 mmol, 0.8 equiv.) and pentane-2,4-dione (12.0 g, 120 mmol, 1 equiv.) in AcOH (274 mL) was added AcONa (39.3 g, 479 mmol, 4 equiv.). The solution was heated to 90 °C and Zn (10.5 g, 161 mmol, 3 equiv.) was added portionwise. The mixture was then heated to 90 °C for 1 h, filtered and evaporated. The residue was dissolved in water and the aqueous phase was extracted three times with EtOAc. The combined organics were washed with brine, dried over $Na_2SO_4$ and evaporated. The residue was purified on silica gel column eluting with 10-50% EtOAc in cyclohexane to give the desired product (20.9 g, 60.7 mmol, 63%) as a colorless solid. $^1$H NMR (400 MHz, $CDCl_3$) δ 9.48 (s, 1H), 7.40 - 7.27 (m, 5H), 4.51 (s, 2H), 4.31 (q, *J* = 7.2 Hz, 2H), 3.55 (t, *J* = 6.6 Hz, 2H), 3.21 - 3.07 (m, 2H), 2.52 (s, 3H), 2.46 (s, 3H), 1.93 - 1.78 (m, 2H), 1.33 (t, *J* = 7.1 Hz, 3H); $^{13}$C NMR (101 MHz, $CDCl_3$) δ 195.3, 161.7, 138.9, 138.0, 134.2, 128.3, 127.6, 127.4, 123.1, 118.0, 72.8, 70.5, 60.5, 31.4, 31.1, 22.7,

15.3, 14.4; HRMS (ESI): calcd. for $C_{20}H_{25}NO_4Na$ $[M+Na]^+$: 366.1676, found : 366.1676; $R_f$: 0.49 (40% EtOAc in cyclohexane (CyH)).

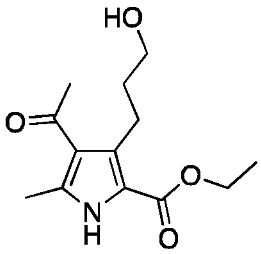

Compound VI

**Ethyl 4-acetyl-3-(3-hydroxypropyl)-5-methyl-1*H*-pyrrole-2-carboxylate (Compound VI).** To a solution of ethyl 4-acetyl-3-(3-(benzyloxy)propyl)-5-methyl-1*H*-pyrrole-2-carboxylate (2.40 g, 6.99 mmol, 1 equiv.) in EtOAc and MeOH (28 mL, 1:1) under argon, was added Pd/C (580 mg, 5% w/w). The solution was put under $H_2$ (1 atm) and stirred overnight at rt. The mixture was filtered over celite, evaporated and purified on silica gel column eluting with 50-100% EtOAc in CyH to afford the desired product (1.19 g, 4.70 mmol, 67%) as a light yellow solid. $^1H$ NMR (400 MHz, $CDCl_3$) $\delta$ 9.92 (s, 1H), 4.31 (q, *J* = 7.2 Hz, 2H), 3.53 (dd, *J* = 6.1, 5.3 Hz, 2H), 3.36 (br s, 1H), 3.15 (t, *J* = 6.9 Hz, 2H), 2.54 (s, 3H), 2.44 (s, 3H), 1.89 - 1.72 (m, 2H), 1.34 (t, *J* = 7.1 Hz, 3H); $^{13}C$ NMR (101 MHz, $CDCl_3$) $\delta$ 195.8, 161.6, 138.1, 134.5, 123.2, 118.4, 61.1, 60.6, 33.5, 31.1, 21.3, 15.5, 14.4; HRMS (ESI) : calcd. for $C_{13}H_{19}NO_4Na$ $[M+Na]^+$: 276.1206, found : 276.1206; $R_f$: 0.42 (80% EtOAc in CyH)

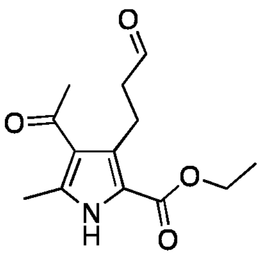

Compound VII

**Ethyl 4-acetyl-5-methyl-3-(3-oxopropyl)-1*H*-pyrrole-2-carboxylate (Compound VII).** To a solution of DMSO (374 μL, 3.16 mmol, 2 equiv.) in dry $CH_2Cl_2$ (10 mL) was added $(COCl)_2$ (203 μL, 2.37 mmol, 1.5 equiv.) at -78 °C dropwise. The mixture was stirred at - 78 °C for 15 min, then a solution of ethyl 4-acetyl-3-(3-hydroxypropyl)-5-methyl-1*H*-pyrrole-2-carboxylate (400 mg, 1.58 mmol, 1 equiv.) in dry $CH_2Cl_2$ (5.8 mL) was added dropwise. After 30 min, $NEt_3$ (1.10 mL, 7.90 mmol, 5 equiv.) was added. The mixture was stirred 30 min at -78 °C, then, was allowed to return to rt. The solution was evaporated and water was added. The aqueous phase was extracted three times with EtOAc and evaporated to afford after fast purification on silica gel column (20-100% EtOAc in cyclohexane), the desired product (397 mg, 1.58 mmol, 100%) as a colorless oil. $^1H$ NMR (400 MHz, $CDCl_3$) $\delta$ 9.81 (t, *J* = 2.0 Hz, 1H), 9.07 (s, 1H), 4.33 (q, *J* = 7.1 Hz, 2H), 3.40 (dd, *J* = 8.0, 7.1 Hz, 2H), 2.74 - 2.65 (m, 2H), 2.44 (s, 3H), 1.35 (t, *J* = 7.1 Hz, 3H); $^{13}C$ NMR (101 MHz, $CDCl_3$) $\delta$ 202.7, 194.7, 161.1, 137.2, 132.8, 123.1, 118.4, 60.7, 44.8, 31.2, 19.1, 15.7, 14.5; HRMS (ESI): calcd. for $C_{13}H_{16}NO_4$ $[M-H]^-$: 250.1085, found : 250.1085; $R_f$: 0.50 (50% AcOEt in CyH)

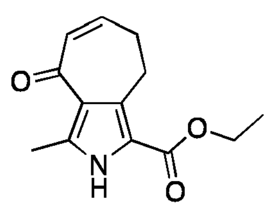

Compound 9

**Ethyl 3-methyl-4-oxo-2,4,7,8-tetrahydrocyclohepta[*c*]pyrrole-1-carboxylate (Compound 9).** To a solution of ethyl 4-acetyl-5-methyl-3-(3-oxopropyl)-1*H*-pyrrole-2-carboxylate (7.30 g, 29.1 mmol, 1 equiv.) in PhMe (291 mL) was added PTSA monohydrate (2.76 g, 14.5 mmol, 0.5 equiv.). The mixture was heated to reflux for 3 h, cooled to rt, evaporated. Concentrated NaHCOs solution was added and the aqueous phase was extracted three times with EtOAc. The combined organics were washed with brine, dried over $Na_2SO_4$ and evaporated. The residue was purified on silica

gel column eluting with 20-50% EtOAc in CyH to afford the desired product (3.79 g, 16.2 mmol, 56%) as a white solid. $^1$H NMR (400 MHz, $CDCl_3$) δ 9.32 (s, 1H), 6.66 (dt, *J* = 12.1, 6.0 Hz, 1H), 6.16 (dt, *J* = 12.3, 1.5 Hz, 1H), 4.35 (q, *J* = 7.1 Hz, 2H), 3.33 - 3.23 (m, 2H), 2.59 (d, *J* = 0.6 Hz, 3H), 2.52 (ddt, *J* = 10.5, 5.9, 1.6 Hz, 2H), 1.39 (t, *J* = 7.1 Hz, 3H); $^{13}$C NMR (101 MHz, $CDCl_3$) δ 188.7, 161.5, 143.8, 140.7, 134.4, 132.5, 123.5, 115.9, 60.5, 28.0, 24.4, 14.8, 14.6; HRMS (ESI) : calcd. for $C_{13}H_{16}NO_3$ $[M+H]^+$: 234.1125, found : 234.1125

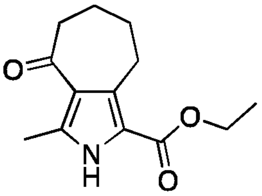

Compound 8

**Ethyl 3-methyl-4-oxo-2,4,5,6,7,8-hexahydrocyclohepta[*c*]pyrrole-1-carboxylate (Compound 8).** To a solution of ethyl 3-methyl-4-oxo-2,4,7,8-tetrahydrocyclohepta[c]pyrrole-1-carboxylate (3.50 g, 15.0 mmol, 1 equiv.) in EtOAc/MeOH (150 mL, 1:1) under argon was added Pd/C (700 mg, 5% w/w). The reaction was stirred at rt for 16h under $H_2$. After filtration and purification on silica gel column (20-60% EtOAc in CyH), to obtain the desired compound as a white solid (3.46 g, 14.7 mmol, 98%). $^1$H NMR (400 MHz, $CDCl_3$) δ 9.62 (s, 1H), 4.32 (q, *J* = 7.1 Hz, 2H), 3.25 - 3.07 (m, 2H), 2.70 - 2.57 (m, 2H), 2.51 (s, 3H), 1.89 - 1.72 (m, 4H), 1.35 (t, *J* = 7.1 Hz, 3H); $^{13}$C NMR (101 MHz, $CDCl_3$) δ 200.0, 161.8, 138.7, 134.2, 123.5, 116.6, 60.4, 42.0, 25.3, 23.4, 21.9, 14.5, 13.8; HRMS (ESI): calcd. for $C_{13}H_{16}NO_3$ $[M-H]^-$: 234.1136, found : 234.1136; $R_f$: 0.47 (30% AcOEt in CyH)

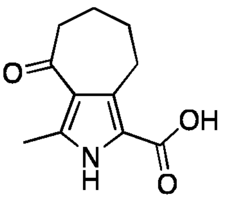

Compound VIII

**3-methyl-4-oxo-2,4,5,6,7,8-hexahydrocyclohepta[*c*]pyrrole-1-carboxylic acid** (Compound **VIII**). To a solution of ethyl 3-methyl-4-oxo-2,4,5,6,7,8-hexahydrocyclohepta[c]pyrrole-1-carboxylate (1.15 g, 4.89 mmol, 1 equiv.) was dissolved in EtOH and aqueous 2M NaOH (48.9 mL, 1:1). The mixture was heated at reflux overnight, cooled at 0 °C and neutralized until pH 1 using concentrated aqueous HCl (12M). The resulting precipitate was filtered and recrystallised using $CHCl_3$ to afford the desired product (723 mg, 3.49 mmol, **71**%) as a white solid. **$^1$H NMR** (500 MHz, DMSO-*d*$_6$) δ 12.44 (s, 1H), 11.81 (s, 1H), 3.13 (td, *J* = 6.2, 5.3, 2.9 Hz, 2H), 2.55 - 2.50 (m, 1H), 2.36 (s, 2H), 1.75 - 1.65 (m, 4H); **$^{13}$C NMR** (126 MHz, DMSO-*d*$_6$) δ 198.4, 162.3, 138.0, 133.0, 122.3, 116.6, 41.3, 24.8, 22.3, 21.2, 13.1; **HRMS** (APCI): calcd. for $C_{11}H_{14}NO_3$ $[M+H]^+$: 208.0968, found : 208.0968; **$R_f$:** 0.63 (100% AcOEt + 2% AcOH).

**Precursor-Example 2. Synthesis of 3-amino-*N,N*-diethyl-4-methoxybenzenesulfonamide**

General Synthetic Scheme

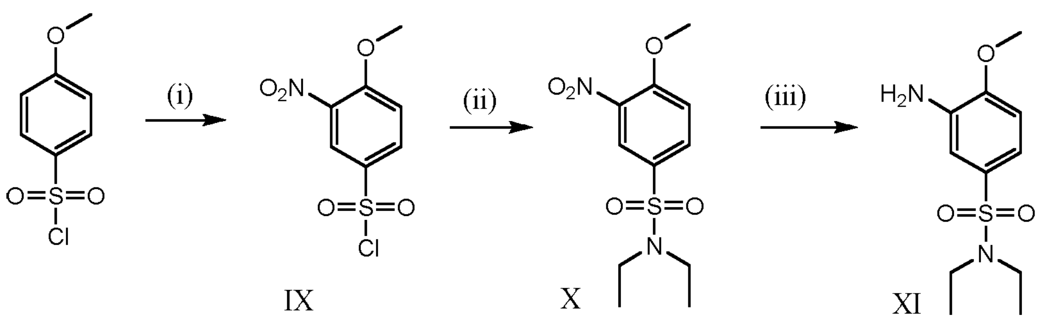


IX X XI

Reaction conditions: i) $HNO_3$, $H_2SO_4$, 0 °C, 60% ; ii) diethylamine ($Et_2NH$), $CH_2Cl_2$, 0 °C to rt, 99% ; iii) $H_2$,

Pd/C, MeOH/AcOEt, rt, 96%

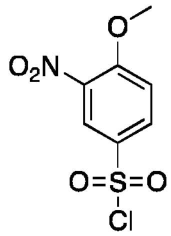

Compound IX

**4-methoxy-3-nitrobenzenesulfonyl chloride** (Compound **IX**). To a solution of 4-methoxybenzenesulfonyl chloride (6.18 g, 29.9 mmol, 1 equiv.) in $H_2SO_4$ (30 mL) at 0 °C, was added $HNO_3$ (1.91 mL, 44.8 mmol, 1.5 equiv.). After 2 h, the solution was poured into ice/water and extracted with $Et_2O$. The organic phase was washed with water, brine, dried over $Na_2SO_4$ and evaporated to afford the desired product (4.53 g, 18.0 mmol, **60**%) as a white solid. **$^1$H NMR** (500 MHz, $CDCl_3$) δ 8.51 (d, *J* = 2.5 Hz, 1H), 8.21 (dd, *J*= 9.0, 2.4 Hz, 1H), 7.31 (d, *J*= 9.1 Hz, 1H), 4.11 (s, 3H); **$^{13}$C NMR** (126 MHz, $CDCl_3$) δ 157.7, 139.2, 135.9, 132.8, 125.5, 114.5, 57.6; **$R_f$:** 0.19 (33% AcOEt in CyH).

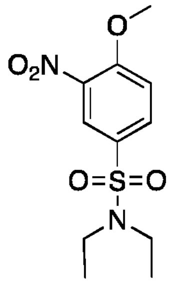

Compound X

***N,N*-diethyl-4-methoxy-3-nitrobenzenesulfonamide** (Compound **X**). To a solution of 4-methoxy-3-nitrobenzenesulfonyl chloride (4.53 g, 18.0 mmol, 1 equiv.) in $CH_2Cl_2$ (36 mL) at 0 °C, was added diethylamine (9.31 mL, 54.0 mmol, 3 equiv.) dropwise. The solution was then stirred at rt for 16 h, washed with 1M HCl, brine, dried over $Na_2SO_4$ and evaporated. The residue was purified on silica gel column eluting with 20-80% EtOAc in CyH to afford the desired product (5.15 g, 17.9 mmol, **99**%) as a light yellow solid. **$^1$H NMR** (500 MHz, $CDCl_3$) δ 8.23 (d, *J* = 2.3 Hz, 1H), 7.95 (dd, *J* = 8.8, 2.3 Hz, 1H), 7.19 (d, *J* = 8.9 Hz, 1H), 4.02 (s, 3H), 3.23 (q, *J* = 7.1 Hz, 4H), 1.13 (t, *J* = 7.2 Hz, 6H); **$^{13}$C NMR** (126 MHz, $CDCl_3$) δ 155.4, 139.2, 132.8, 132.7, 124.8, 114.0, 57.1, 42.2, 14.2; **HRMS** (ESI) : calcd. for $C_{11}H_{17}N_2O_5$ $[M+H]^+$: 289.0855, found: 289.0855; **$R_f$:** 0.40 (100% $CH_2Cl_2$); **$R_f$:** 0.38 (60% AcOEt in CyH)

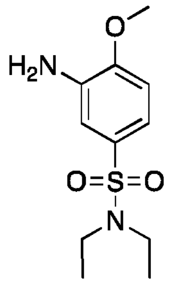

Compound XI

**Example 3. Synthesis of *N*-(5-(*N,N*-diethylsulfamoyl)-2-methoxyphenyl)-3-methyl-4-oxo-2,4,5,6,7,8-hexahydrocyclohepta[c]pyrrole-1-carboxamide**

General Synthetic Scheme

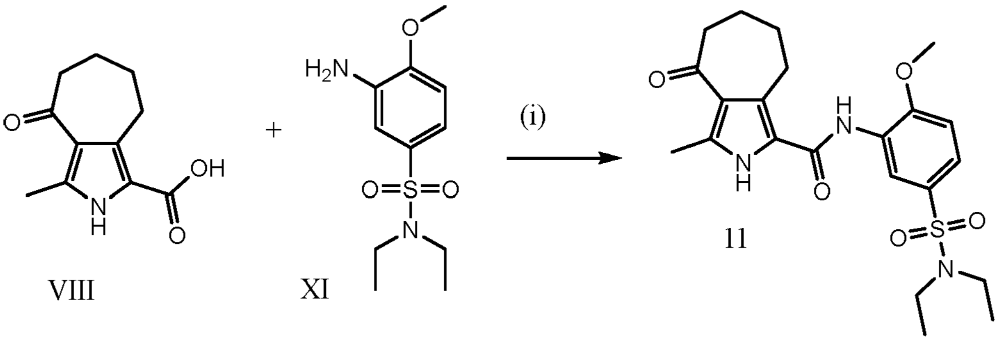


VIII XI 11

Reaction conditions: i) HCTU, DIEA, DMF, 0 °C to 100 °C, 64%

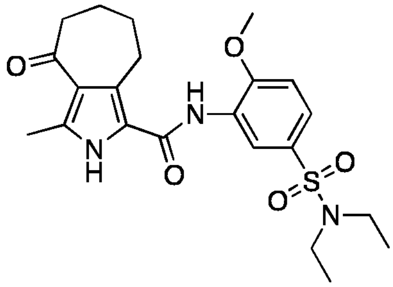

Compound 11

***N*-(5-(*N*,*N*-diethylsulfamoyl)-2-methoxyphenyl)-3-methyl-4-oxo-2,4,5,6,7,8-hexahydrocyclohepta[*c*]pyrrole-1-carboxamide** (Compound **11**). To a solution of 3-methyl-4-oxo-2,4,5,6,7,8-hexahydrocyclohepta[*c*]pyrrole-1-carboxylic acid (100 mg, 0.48 mmol, 1 equiv.) and 3-amino-*N*,*N*-diethyl-4-methoxybenzenesulfonamide (155 mg, 0.60 mmol, 1.25 equiv.) in dry DMF (1.9 mL) at - 0 °C, were added diisopropylethylamine (DIEA) (0.42 mL, 2.41 mmol, 5 equiv.) and HCTU (2-(6-Chloro-1h-benzotriazol-1-yl),1,1,3,3-tetramethylaminium-hexafluorophopsphate) (250 mg, 0.60 mmol, 1.25 equiv.). The reaction was allowed to warm at rt. After 4 h, the mixture was heated at 100 °C for 16 h. Then, DMF was evaporated. Water was added and the aqueous phase was extracted three times with EtOAc. The combined organics were washed with 1H HCl, saturated $Na_2CO_3$, brine, dried over $Na_2SO_4$. Purification on silica gel column eluting with 20-100% EtOAc in cyclohexane to afford the desired product (139 mg, 0.31 mmol, **64**%) as a light yellow solid. **$^1$H NMR** (400 MHz, $CDCl_3$) $\delta$ 10.13 (s, 1H), 8.84 (d, *J* = 2.3 Hz, 1H), 8.22 (s, 1H), 7.52 (dd, *J* = 8.5, 2.3 Hz, 1H), 6.93 (d, *J* = 8.7 Hz, 1H), 3.98 (s, 3H), 3.22 (q, *J* = 7.1 Hz, 4H), 3.15 - 3.06 (m, 2H), 2.72 - 2.62 (m, 2H), 2.49 (d, *J* = 0.5 Hz, 3H), 2.00 - 1.80 (m, 4H), 1.11 (t, *J* = 7.2 Hz, 6H) ; **$^{13}$C NMR** (101 MHz, $CDCl_3$) $\delta$ 199.5, 159.8, 150.6, 138.6, 132.8, 128.1, 127.9, 123.5, 123.2, 120.7, 118.1, 109.6, 56.5, 42.2, 41.9, 25.8, 24.5, 21.8, 14.3, 13.7; **HRMS** (ESI) : calcd. for $C_{22}H_{29}N_3O_5SNa$ $[M+Na]^+$: 470.1720, found: 470.1720; **$R_f$:** 0.57 (66% AcOEt in CyH)

**Precursor-Example 4.** Synthesis of 2-methoxy-5-(morpholinosulfonyl)aniline

**General Synthetic Scheme**

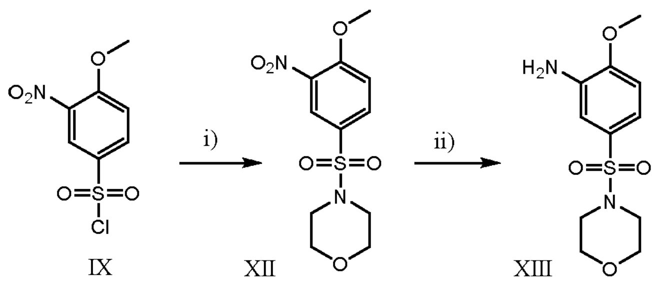


IX XII XIII

Reaction conditions: i) Morpholine, $CH_2Cl_2$, 0 °C to rt, 87% ; ii) $H_2$, Pd/C, MeOH/AcOEt, rt, 91%

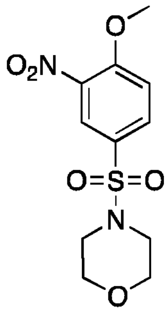

Compound XII

**4-((4-methoxy-3-nitrophenyl)sulfonyl)morpholine** (Compound XII). The compound was synthesized as reported for *N*,*N*-diethyl-4-methoxy-3-nitrobenzenesulfonamide (morpholine was used instead of diethylamine), to afford the desired compound (3.14 g, 10.4 mmol, **87**%) as a light yellow solid.

$^{1}$**H NMR** (400 MHz, DMSO-$d_6$) δ 8.20 (d, *J* = 2.3 Hz, 1H), 8.00 (dd, *J* = 8.9, 2.3 Hz, 1H), 7.61 (d, *J* = 9.0 Hz, 1H), 4.04 (s, 3H), 3.68 - 3.60 (m, 4H), 2.96 - 2.88 (m, 4H); $^{13}$**C NMR** (101 MHz, DMSO) δ 155.2, 138.9, 133.4, 126.3, 124.6, 115.4, 65.2, 57.4, 45.7; **HRMS** (ESI) : calcd. for $C_{11}H_{14}N_2O_6SNa$ $[M+Na]^+$: 325.0465, found: 325.0465; **R$_f$:** 0.35 (60% AcOEt in CyH)

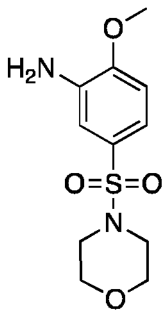

Compound XIII

**2-methoxy-5-(morpholinosulfonyl)aniline** (Compound **XIII**). The compound was synthesized as reported for 3-amino-*N*,*N*-diethyl-4-methoxybenzenesulfonamide in MeOH/EtOAc (1:1, 50 mL), to afford the desired compound (1.89 g, 6.94 mmol, **91**%) as a light yellow solid. $^{1}$**H NMR** (500 MHz, DMSO-$d_6$) δ 7.04 - 6.94 (m, 2H), 6.88 (dt, *J* = 8.4, 2.1 Hz, 1H), 3.84 (d, *J* = 1.6 Hz, 3H), 3.60 (t, *J* = 4.8 Hz, 4H), 2.84 - 2.74 (m, 4H); $^{13}$**C NMR** (126 MHz, DMSO-$d_6$) δ 149.6, 138.3, 125.8, 116.2, 111.5, 109.9, 65.3, 55.6, 45.9; **HRMS** (ESI) : calcd. for $C_{11}H_{16}N_2O_4SNa$ $[M+Na]^+$: 295.0723, found: 295.0723; **R$_f$:** 0.72 (80% AcOEt in CyH)

**Example 5. Synthesis of *N*-(2-methoxy-5-(morpholinosulfonyl)phenyl)-3-methyl-4-oxo-2,4,5,6,7,8-hexahydrocyclohepta[*c*]pyrrole-1-carboxamide**

General Synthetic Scheme

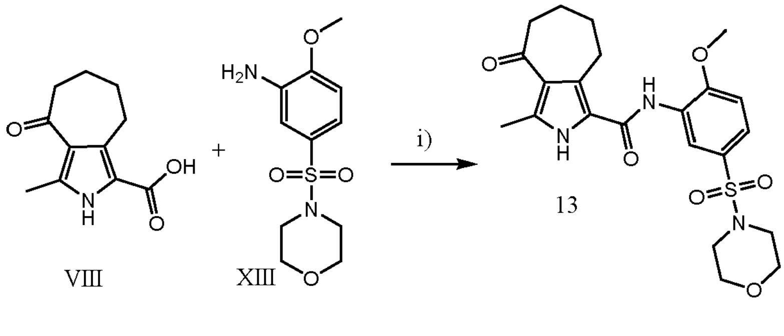


VIII XIII 13

Reaction conditions: i) O-(1*H*-6-Chlorobenzotriazole-1-yl)-1,1,3,3-tetramethyluronium hexafluorophosphate (HCTU), *N*,*N*-Diisopropylethylamine (DIEA), DMF, 0 °C to 100 °C, 38%

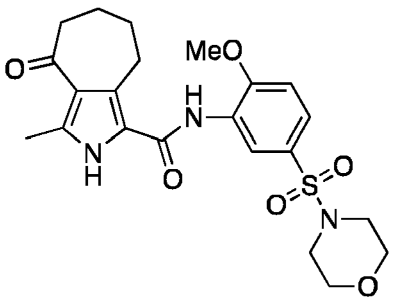

Compound 13

***N*-(2-methoxy-5-(morpholinosulfonyl)phenyl)-3-methyl-4-oxo-2,4,5,6,7,8-hexahydrocyclohepta[*c*]pyrrole-1-carboxamide**. The compound was synthesized as reported for *N*-(5-(*N*,*N*-diethylsulfamoyl)-2-methoxyphenyl)-3-methyl-4-oxo-2,4,5,6,7,8-hexahydrocyclohepta[c]-pyrrole-1-carboxamide, to afford the desired compound (84 mg, 0.18 mmol, **38**%) as a light yellow solid. **$^1$H NMR** (400 MHz, $CDCl_3$) δ 9.90 (s, 1H), 8.83 (d, *J* = 2.3 Hz, 1H), 8.25 (s, 1H), 7.48 (dd, *J* = 8.6, 2.3 Hz, 1H), 7.01 (d, *J* = 8.7 Hz, 1H), 4.02 (s, 3H), 3.77 - 3.65 (m, 4H), 3.13 - 3.07 (m, 2H), 3.05 - 2.98 (m, 4H), 2.71 - 2.66 (m, 2H), 2.50 (s, 3H), 2.01 - 1.85 (m, 4H); **$^{13}$C NMR** (101 MHz, $CDCl_3$) δ 199.5, 159.8, 151.1, 138.7, 128.3, 127.8, 127.7, 124.0, 123.6, 120.7, 118.9, 109.8, 66.2, 56.6, 46.2, 41.9, 25.9, 24.7, 21.8, 13.8; **HRMS** (ESI) : calcd. for $C_{22}H_{27}N_3O_6SNa$ $[M+Na]^+$: 484.1513, found: 484.1513; **$R_f$:** 0.19 (66% AcOEt in CyH)

**Precursor-Example 6. Synthesis of 2-methoxy-5-((4-methylpiperazin-1-yl)sulfonyl)aniline**

General Synthetic Scheme

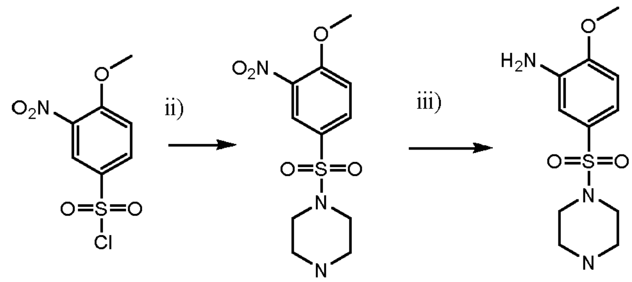


IX XIV XV

Reaction conditions: i) *N*-Methylpiperazine, $CH_2Cl_2$, 0 °C to rt, 87% ; ii) $H_2$, Pd/C, MeOH/AcOEt, rt, 91%

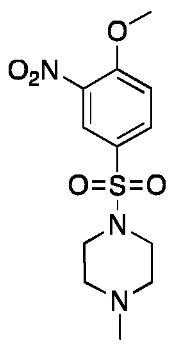

Compound XV

**2-methoxy-5-((4-methylpiperazin-1-yl)sulfonyl)aniline** (Compound **XV**). The compound was synthesized

as reported for 3-amino-*N*,*N*-diethyl-4-methoxybenzenesulfonamide in MeOH/EtOAc (1:1, 50 mL), to afford the desired compound (1.66 g, 5.82 mmol, **92**%) as a light yellow solid. **HRMS** (ESI) : calcd. for $C_{12}H_{20}N_3O_3S$ [M+H]$^+$: 286.1220, found: 286.1220

**Example 7. Synthesis of N-(2-methoxy-5-((4-methylpiperazin-1-yl)sulfonyl)phenyl)-3-methyl-4-oxo-2,4,5,6,7,8-hexahydrocyclohepta[c]pyrrole-1-carboxamide**

General Synthetic Scheme

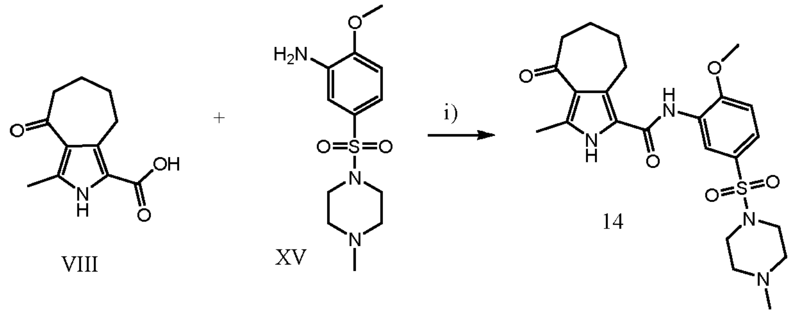

VIII
XV
14

Reaction conditions: i) HCTU, DIEA, DMF, 0 °C to 100 °C, 41%

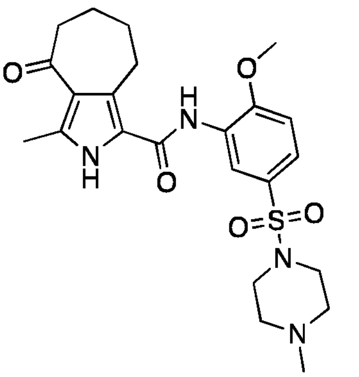

Compound 14

***N*-(2-methoxy-5-((4-methylpiperazin-1-yl)sulfonyl)phenyl)-3-methyl-4-oxo-2,4,5,6,7,8-hexahydrocyclohepta[*c*]pyrrole-1-carboxamide** (Compound **14**). The compound was synthesized as reported for *N*-(5-(*N*,*N*-diethylsulfamoyl)-2-methoxyphenyl)-3-methyl-4-oxo-2,4,5,6,7,8-hexahydrocyclohepta[c]pyrrole-1-carboxamide, to afford the desired compound (94 mg, 0.20 mmol, **41**%) as a light yellow solid. **$^1$H NMR** (400 MHz, $CDCl_3$) δ 9.98 (s, 1H), 8.83 (d, *J* = 2.3 Hz, 1H), 8.23 (s, 1H), 7.47 (dd, *J* = 8.6, 2.3 Hz, 1H), 6.98 (d, *J* = 8.7 Hz, 1H), 4.00 (s, 3H), 3.14 - 2.99 (m, 6H), 2.73 - 2.62 (m, 2H), 2.49 (d, *J* = 0.4 Hz, 3H), 2.45 (t, *J* = 5.0 Hz, 4H), 2.24 (s, 3H), 2.00 - 1.81 (m, 4H); **$^{13}$C NMR** (101 MHz, $CDCl_3$) δ 199.5, 159.7, 151.0, 138.6, 128.3, 127.9, 127.7, 123.9, 123.6, 120.8, 118.8, 109.7, 56.6, 54.2, 46.1, 45.8, 41.9, 25.9, 24.6, 21.8, 13.8; **HRMS** (ESI) : calcd. for $C_{23}H_{31}N_4O_5S$ [M+H]$^+$: 475.2010, found: 475.2010; **$R_f$**: 0.05 (66% AcOEt in CyH); **$R_f$**: 0.36 (12% MeOH in $CH_2Cl_2$ + 0.1% $NEt_3$)

**Precursor-Example 8. Synthesis of 5-(azepan-1-ylsulfonyl)-2-methoxyaniline**

General Synthetic Scheme

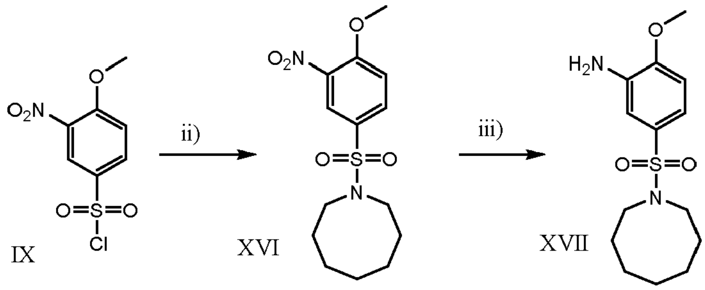

IX XVI XVII

Reaction conditions: i) Azepane, $CH_2Cl_2$, 0 °C to rt, 94% ; ii) $H_2$, Pd/C, MeOH/AcOEt, rt, 95%

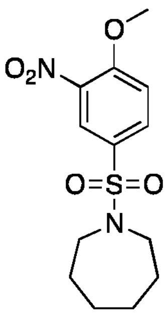
Compound XVI

**1-((4-methoxy-3-nitrophenyl)sulfonyl)azepane** (Compound **XVI**). The compound was synthesized as reported for *N*,*N*-diethyl-4-methoxy-3-nitrobenzenesulfonamide (azepane was used instead of diethylamine), to afford the desired compound (3.52 g, 11.2 mmol, **94**%) as a light yellow solid. **$^1$H NMR** (500 MHz, $CDCl_3$) $\delta$ 8.23 (d, *J* = 2.3 Hz, 1H), 7.95 (dd, *J* = 8.8, 2.3 Hz, 1H), 7.19 (d, *J* = 8.9 Hz, 1H), 4.03 (s, 3H), 3.32 - 3.19 (m, 4H), 1.72 (ddddd, *J* = 9.9, 8.1, 6.7, 3.3, 2.2 Hz, 4H), 1.63 - 1.53 (m, 4H); **$^{13}$C NMR** (126 MHz, $CDCl_3$) $\delta$ 155.4, 139.3, 132.6, 132.0, 124.7, 113.9, 57.1, 48.4, 29.2, 26.9; **HRMS** (ESI) : calcd. for $C_{13}H_{19}N_2O_5S$ $[M+H]^+$: 315.1009, found: 315.1009; **$R_f$:** 0.56 (60% AcOEt in CyH)

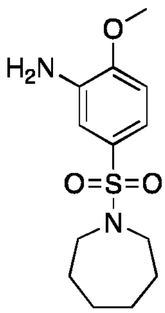
Compound XVII

**5-(azepan-1-ylsulfonyl)-2-methoxyaniline** (Compound **XVII**). The compound was synthesized as reported for 3-amino-*N,N*-diethyl-4-methoxybenzenesulfonamide in MeOH/EtOAc (1:1, 50 mL), to afford the desired compound (1.72 g, 6.05 mmol, 95%) as a light yellow solid. $^1$H **NMR** (400 MHz, DMSO-$d_6$) $\delta$ 8.85 (s, 3H), 7.68 (d, *J* = 2.3 Hz, 1H), 7.50 (dd, *J* = 8.6, 2.4 Hz, 1H), 7.22 (dd, *J* = 8.7, 1.1 Hz, 1H), 3.91 (s, 3H), 3.24 - 3.12 (m, 4H), 1.72 - 1.42 (m, 8H); **$^{13}$C NMR** (101 MHz, DMSO-$d_6$) $\delta$ 153.2, 131.0, 127.0, 123.8, 118.8, 111.8, 56.3, 47.6, 28.4, 26.3; **HRMS**

(ESI) : calcd. for $C_{13}H_{21}N_2O_3S$ $[M+H]^+$: 285.1287, found: 285.1287; **$R_f$:** 0.48 (40% AcOEt in PE)

**Example 9. Synthesis of *N*-(5-(azepan-1-ylsulfonyl)-2-methoxyphenyl)-3-methyl-4-oxo-2,4,5,6,7,8-hexahydrocyclohepta[*c*]pyrrole-1-carboxamide**

General Synthetic Scheme

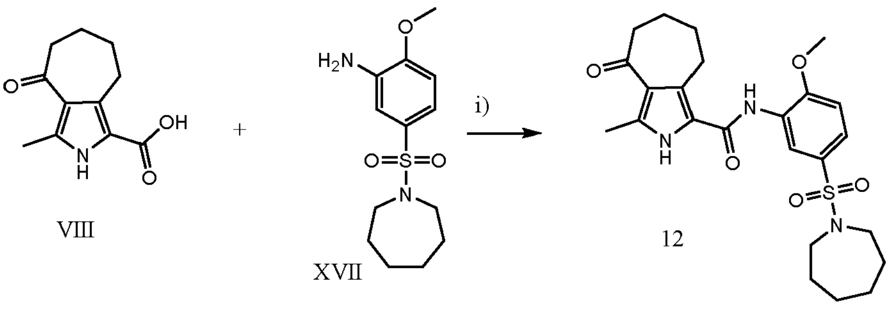


VIII

XVII

12

Reaction conditions: i) HCTU, DIEA, DMF, 0 °C to 100 °C, 73%

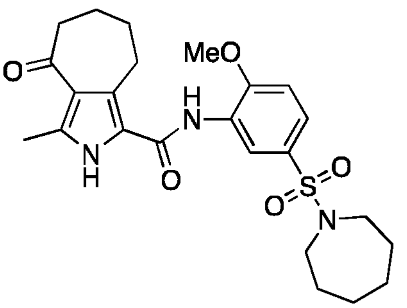

Compound 12

***N*-(5-(azepan-1-ylsulfonyl)-2-methoxyphenyl)-3-methyl-4-oxo-2,4,5,6,7,8-hexahydrocyclohepta[*c*]pyrrole-1-carboxamide** (Compound **12**). The compound was synthesized as reported for *N*-(5-(*N*,*N*-diethylsulfamoyl)-2-methoxyphenyl)-3-methyl-4-oxo-2,4,5,6,7,8-hexahydrocyclohepta[c]pyrrole-1-carboxamide, to afford the desired compound (167 mg, 0.35 mmol, **73**%) as a light yellow solid. **$^1$H NMR** (400 MHz, $CDCl_3$) δ 10.07 (s, 1H), 8.83 (d, *J* = 2.3 Hz, 1H), 8.23 (s, 1H), 7.51 (dd, *J* = 8.6, 2.3 Hz, 1H), 6.94 (d, *J* = 8.6 Hz, 1H), 3.99 (s, 3H), 3.31 - 3.23 (m, 4H), 3.12 - 3.07 (m, 2H), 2.72 - 2.63 (m, 2H), 2.49 (d, *J* = 0.5 Hz, 3H), 1.99 - 1.82 (m, 4H), 1.68 (dtt, *J* = 10.9, 4.7, 2.4 Hz, 4H), 1.61 - 1.52 (m, 4H); **$^{13}$C NMR** (101 MHz, $CDCl_3$) δ 199.5, 159.8, 150.6, 138.6, 132.1, 128.1, 127.9, 123.5, 123.1, 120.8, 117.9, 109.6, 56.5, 48.4, 41.9, 29.2, 27.0, 25.8, 24.5, 21.8, 13.7; **HRMS** (ESI) : calcd. for $C_{24}H_{31}N_3O_5SNa$ $[M+Na]^+$: 496.1877, found: 496.1877; **$R_f$:** 0.40 (66% AcOEt in CyH).

**Example 10 - Synthesis of compound 23**

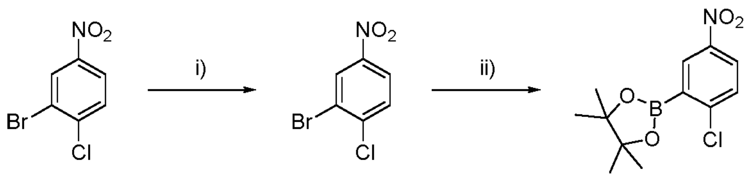


**2-Bromo-1-chloro-4-nitrobenzene.**

NBS (14.1g, 79.2 mmol, 1.2equiv) were added portionwise to a solution of 4-chloronitrobenzene (10.4 g, 66.0 mmol, 1.0 equiv) in conc. $H_2SO_4$ (30 mL over 45 min at 60°C. After 2 h the reaction mixture was poured on ice. The precipitated solids were filtered off, washed first with $H_2O$ and then with pentane. The remaining solid was dried under reduced pressure to obtain the desired product as a yellowish solid (11.3 g, 47.7 mmol, **72%**). **Mp:** 53-54 °C.

**$^1$H-NMR (400 MHz, $CDCl_3$):** δ **=** 7.63 (d, *J* = 8.8 Hz, 1H), 8.12 (dd, *J*= 8.8 Hz, *J*= 2.6 Hz, 1H), 8.50 (d, *J* = 2.6 Hz, 1H). **$^{13}$C-NMR (101 MHz, $CDCl_3$):** δ **=** 123.3, 123.4, 127.5, 128.9, 130.9, 141.9.

**2-(2-Chloro-5-nitrophenyl)-4,4,5,5-tetramethyl-1,3,2-dioxaborolane.** A suspension of 2-(3,5-dinitrophenyl)-4,4,5,5-tetramethyl-1,3,2-dioxaborolane (100 mg, 423 mmol, 1.0 equiv), KOAc (81 mg, 825 mmol, 2.0 equiv.), $B_2(pin)_2$ (120 mg, 465 mmol, 1.1 equiv.), $Pd(dppf)Cl_2$ (9.3 mg, 12.7 mmol, 3 mol%) in degassed dioxane (1.2 mL) and DMSO (20 μL) was stirred overnight at 90 °C. The solvent was removed under reduced pressure and an aqueous solution of

NaOH (2M) was added, the mixture was stirred for 10 min, filtered and washed two times with $Et_2O$. The aqueous phase was acidified with conc. HCl and afterwards three times extracted with EA. The organic phase was dried over $Na_2SO_4$ and evaporated to obtain the title compound as a yellowish solid (63.1 mg, 0.222 mmol, 52%). **Mp:** 89-92 °C.

**$^1$H-NMR (500 MHz, $CDCl_3$):** $\delta$ **=** 1.39 (s, 14H), 7.51 (d, *J* = 8.8 Hz, 1H), 8.18 (dd, *J* = 8.8, 2.8 Hz, 1H), 8.55 (d, *J* = 2.9 Hz, 1H). **$^{13}$C-NMR (101 MHz, $CDCl_3$):** $\delta$ **=** 25.0, 85.1, 126.6, 130.6, 131.5, 132.6, 146.1, 146.7. **HRMS (neg. APCI): $[M + O_2]^-$** calc. for $C_{12}H_{15}NO_6BCl$: 315.0686; found: 315.0688.

**1-bromo-3-methyl-5,6,7,8-tetrahydrocyclohepta[c]pyrrol-4(2*H*)-one**

**General synthetic scheme**

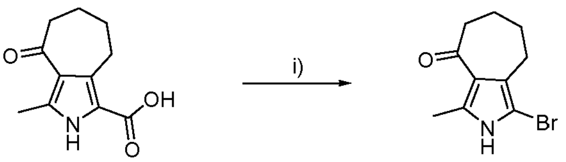


Pyridine (0.54 mL, 6.76 mmol, 5 equiv.) followed by pyridinium tribromide (648 mg, 2.03 mmol, 1.5 equiv.) were added to a solution of 3-methyl-4-oxo-2,4,5,6,7,8-hexahydrocyclohepta[*c*]pyrrole-1-carboxylic acid (280 mg, 1.35 mmol, 1 equiv.) in $CH_2Cl_2$ (13.5 mL) at 0 °C. The mixture was allowed to return to room temperature and stirred overnight. Aqueous hydrochloric acid (HCl, 0.5M) was added and the organic phase was collected, dried over $Na_2SO_4$ and evaporated. Purification on flash chromatography column afford the desired product (159 mg, 0.66 mmol, **49**%) as a colourless solid, which decomposes at room temperature.

**$^1$H NMR** (400 MHz, $CDCl_3$) $\delta$ 9.67 (s, 1H), 2.71 - 2.64 (m, 4H), 2.49 (d, *J* = 0.5 Hz, 3H), 1.92 - 1.70 (m, 4H); **$^{13}$C NMR** (101 MHz, $CDCl_3$) $\delta$ 200.1, 137.2, 123.9, 121.1, 96.0, 41.8, 25.1, 24.2, 22.3, 13.9, **HRMS** (ESI) : calcd. for $C_{10}H_{13}NOBr$ $[M+H]^+$: 242.0175, found : 242.0175; **$R_f$:** 0.45 (33% AcOEt in cyclohexane).

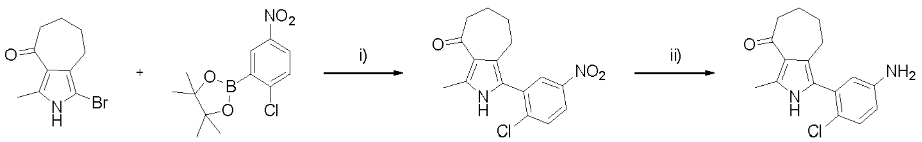


**1-(2-Chloro-5-nitrophenyl)-3-methyl-5,6,7,8-tetrahydrocyclohepta[*c*]pyrrol-4(2*H*)-one.**

A mixture of 1-bromo-3-methyl-5,6,7,8-tetrahydrocyclohepta[c]pyrrol-4(2*H*)-one (73 mg, 0.30 mmol, 1.0 equiv.), 2-(2-Chloro-5-nitrophenyl)-4,4,5,5-tetramethyl-1,3,2-dioxaborolane (0.13 g, 0.45 mmol, 1.5 equiv.), 1,3,5,7-tetramethyl-6-phenyl-2,4,8-trioxa-6-phosphaadamantane (18 mg, 0.06 mmol, 0.02 equiv.), $Pd_2dba_3$ (14 mg, 15 $\mu$mol, 0.05 equiv.), $K_3PO_4$ (0.19 g, 0.90 mmol, 3.0 equiv.) in degassed dioxane/$H_2O$ (1.5 mL) was stirred 3.75 h at 70 °C, then filtered over celite and evaporated. The residue was purified by column chromatography (10-85% EA in PE) to afford the desired product (34 mg, 0.11 mmol, **35**%) as a light yellow solid. **Mp:** 198-199 °C.

**$^1$H-NMR (500 MHz, $CDCl_3$):** $\delta$ **=** 1.80-1.95 (m, 4H), 2.58 (s, 3H), 2.67-2.73 (m, 4H), 7.64 (d, *J* = 8.8 Hz, 1H), 8.12 (d, *J* = 8.8 Hz, *J* = 2.7 Hz, 1H), 8.19 (d, *J* = 2.7 Hz, 1H), 8.38 (br. s, 1H). **$^{13}$C-NMR (126 MHz, $CDCl_3$):** $\delta$ **=** 14.0, 22.2, 23.9, 25.6, 42.0, 121.0, 122.0, 123.3, 125.7, 126.9, 131.3, 132.7, 136.2, 140.2, 146.6, 200.1. **HRMS (pos. ESI): $[M + H]^+$** calc. for $C_{16}H_{15}O_3N_2Cl$: 319.0844; found: 319.0842.

**1-(5-Amino-2-chlorophenyl)-3-methyl-5,6,7,8-tetrahydrocyclohepta[*c*]pyrrol-4(2*H*)-one (compound 23)**

To a solution of 1-(2-chloro-5-nitrophenyl)-3-methyl-5,6,7,8-tetrahydrocyclohepta[*c*]pyrrol-4(2*H*)-one (33 mg, 0.11 mmol, 1.0 equiv.) and $NH_4Cl$ (12 mg, 0.23 mmol, 2.0 equiv.) in EtOH/$H_2O$ (4:1, 2.3 mL) Fe powder (22 mg, 0.40 mmol, 3.5 equiv.) was added. The mixture was stirred under reflux for 4 h, afterwards filtered first over cotton, then celite. EtOH was evaporated, water was added and the aqueous phase was three times extracted with EA. The combined organic phases were washed with brine, dried over $Na_2SO_4$ and evaporated. The residue was purified by reversed phase column chromatography (MeCN/$H_2O$) to afford the desired product as a colourless solid (23 mg, 0.79 mmol, **69**%). **Mp:** 169-171 °C.

**$^1$H-NMR (500 MHz, $CDCl_3$):** $\delta$ **=** 1.79-1.92 (m, 4H), 2.54 (d, *J*= 0.5 Hz, 3H), 2.63-2.75 (m, 4H), 3.60-3.83 (m, 2H), 6.54-6.62 (m, 2H), 7.18-7.23 (m, 1H), 8.27 (s, 1H). **$^{13}$C-NMR (126 MHz, $CDCl_3$):** $\delta$ **=** 13.9, 22.2, 23.8, 25.7, 42.0, 115.7, 118.3, 121.5, 122.5, 123.4, 123.6, 130.8, 131.5, 134.7, 145.2, 220.3. **HRMS (pos. ESI): $[M + H]^+$** calc. for $C_{16}H_{18}ON2Cl$: 289.1102; found: 289.1101.

**Example 11 - Synthesis of compound (24)**

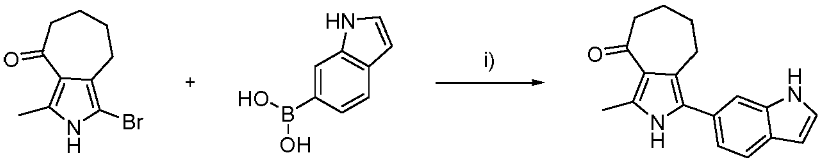


**1-(1*H*-indol-6-yl)-3-methyl-5,6,7,8-tetrahydrocyclohepta[c]pyrrol-4(2*H*)-one (compound 24).**

To 1-bromo-3-methyl-5,6,7,8-tetrahydrocyclohepta[*c*]pyrrol-4(2*H*)-one (50 mg, 0.21 mmol, 1 equiv.), (1*H* indol-6-yl)boronic acid (50 mg, 0.31 mmol, 1.5 equiv.), 1,3,5,7-tetramethyl-6-phenyl-2,4,8-trioxa-6-phosphaadamantane (24 mg, 0.02 mmol, 0.1 equiv.), $Pd_2dba_3$ (19 mg, 0.02 mmol, 0.1 equiv.), $K_3PO_4$ (131 mg, 0.62 mmol, 3 equiv.) degassed dioxane/$H_2O$ (2.1 mL) under argon was added. The mixture was stirred at 60 °C for 16 h, then filtered on celite and evaporated. The mixture was purified on silica gel column eluting with 30-70% EtOAc in PE to afford the desired product (37 mg, 0.13 mmol, **64**%) as a light yellow solid.

**$^1$H NMR** (500 MHz, MeOD-$d_4$) $\delta$ 7.58 (dd, *J* = 8.2, 0.7 Hz, 1H), 7.45 - 7.36 (m, 1H), 7.20 (d, *J* = 3.1 Hz, 1H), 7.08 (dd, *J* = 8.2, 1.5 Hz, 1H), 6.45 (dd, *J* = 3.2, 0.9 Hz, 1H), 2.90 - 2.84 (m, 2H), 2.67 - 2.62 (m, 2H), 2.49 (s, 3H), 1.90 - 1.72 (m, 4H); **$^{13}$C NMR** (126 MHz, MeOD-*$d_4$*) $\delta$ 202.4, 136.7, 135.9, 128.9, 127.2, 126.4, 125.5, 121.9, 121.2, 120.7, 120.1, 110.7, 101.9, 41.8, 26.3, 23.8, 22.7, 13.5; **$R_f$:** 0.25 (40% AcOEt in cyclohexane).

**Example 12** - **Synthesis of compounds (26) and (27)**

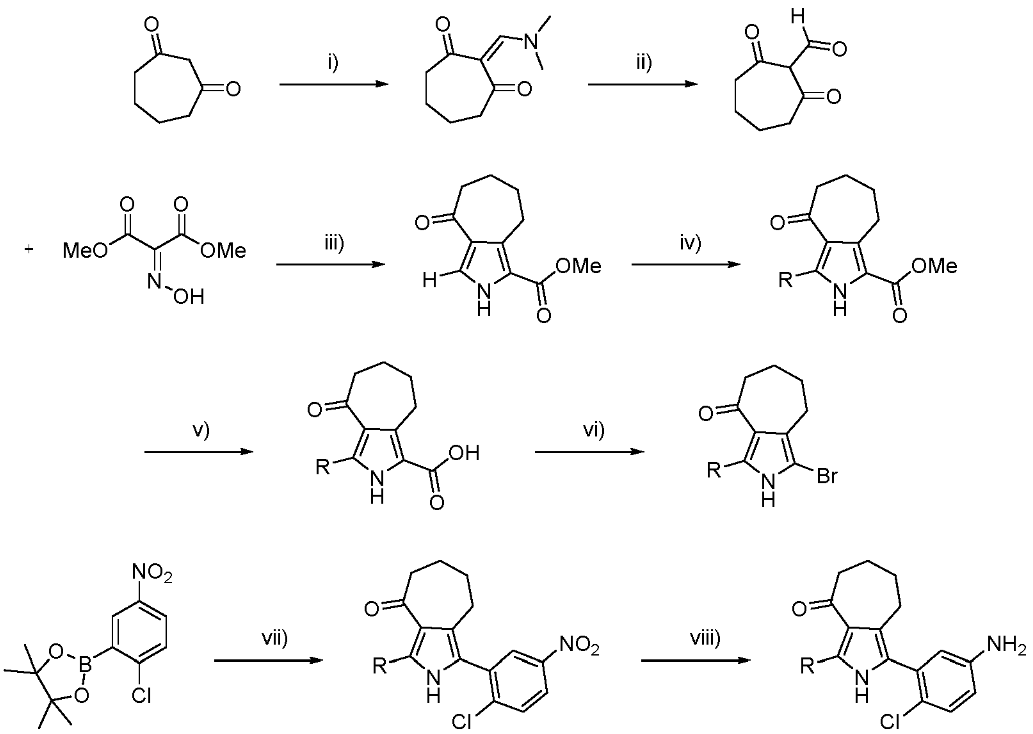

**2-((Dimethylamino)methylene)cycloheptane-1,3-dione (step i).**

To a solution of cycloheptane-1,3-dione (7.61 g, 60.3 mmol, 1.0 equiv.) in dry DCM (86 mL) *N*,*N*-dimethylformamide dimethyl acetal (10.5 mL, 9.34 g, 78.4 mmol, 1.3 equiv.) was added. The mixture was stirred at room temperature overnight, evaporated and purified by column chromatography (8% MeOH in DCM) to afford the desired product (9.49 g, 52.4 mmol, **87**%) as a yellow solid. **Mp:** 91-92 °C.

**$^1$H-NMR (400 MHz, $CDCl_3$): δ =** 1.83 ($m_c$, 4H), 2.56 ($m_c$, 4H), 2.78 (s, 3H), 3.27 (s, 3H), 7.69 (s, 1H). **$^{13}$C-NMR (101 MHz, $CDCl_3$):** δ = 22.3, 40.6, 43.3, 48.0, 112.9, 159.6, 200.1

**2,7-Dioxocycloheptane-1-carbaldehyde (step ii).**

To a solution of 2-((dimethylamino)methylene)-cycloheptane-1,3-dione (9.49 g, 52.3 mmol, 1.0 equiv.) in THF (70 mL) aqueous HCl (1%, 260 mL) was added. The solution was stirred for 3 h at room temperature, THF was removed and the aqueous phase was extracted three times with EA. The combined organic phases were washed with water, brine, dried over $Na_2SO_4$ and evaporated to afford the desired product (7.52 g, 48.8 mmol, **93**%) as a light orange oil. **$^1$H-NMR (500 MHz, $CDCl_3$): δ =** 1.86-1.95 (m, 4H), 2.64-2.70 (m, 2H), 2.74-2.78 (m, 2H), 9.29 (br. s, 1H). **$^{13}$C-NMR (126 MHz, $CDCl_3$): δ =** 21.2, 22.1, 36.2, 41.1, 117.3, 190.3, 198.4, 199.2. **HRMS (neg. APCI): [M - H]-** calc. for $C_8H_9O_3$: 153.0557; found: 153.0558.

**Methyl 4-oxo-2,4,5,6,7,8-hexahydrocyclohepta[*c*]pyrrole-1-carboxylate (step iii)**

Dimethyl 2-(hydroxy-imino)malonate (10.2 g, 63.5 mmol, 1.35 equiv), 2,7-dioxocycloheptane-1-carbaldehyde (7.25 g, 47.0 mmol, 1.0 equiv) and NaOAc (13.5 g, 165 mmol, 3.5 equiv.) were dissolved in AcOH (157 mL) and heated to 90 °C. Zn (12.3 g, 188 mmol, 4.0 equiv) was added portionwise. The reaction mixture was stirred for 5 h at 95 °C, poured on ice and the aqueous phase was extracted three times with EA. The combined organic layers were washed twice with NaOH, then brine, dried over $Na_2SO_4$ and evaporated. The residue was purified by column chromatography (33-40% EA in PE) to afford the desired product as a colourless solid (2.64 g, 12.8 mmol, **27**%). **Mp:** 129-132 °C.

**$^1$H-NMR (400** MHz, **$CDCl_3$):** δ = 1.80-1.97 (m, 4H), 2.69 ($m_c$, 2H), 3.19 ($m_c$, 2H), 3.87 (s, 3H), 7.50 (d, *J* = 3.5 Hz, 1H), 9.62 (br. s, 1H). **$^{13}$C-NMR (101 MHz, $CDCl_3$):** δ = 22.2, 24.4, 25.9, 42.2, 51.6, 119.6, 126.0, 127.9, 132.9, 161.9, 199.2. **HRMS (pos. APCI): [M + H]$^+$** calc. for $C_{11}H_{13}NO_3$: 208.0968; found: 208.0969.

**Methyl 4-oxo-3-propyl-2,4,5,6,7,8-hexahydrocyclohepta[c]pyrrole-1-carboxylate and Methyl 3-butyl-4-oxo-2,4,5,6,7,8-hexahydrocyclohepta[c]pyrrole-1-carboxylate (step iv).**

Methyl 4-oxo-2,4,5,6,7,8-hexahydrocyclohepta[c]pyrrole-1-carboxylate (0.95 g, 4.6 mmol, 1.0 equiv), 1-bromopropane (0.83 mL, 1.1 g, 9.1 mmol, 2.0 equiv), norbornene (0.86 g, 9.1 mmol, 2.0 equiv), $KHCO_3$ (1.4 g, 14 mmol, 3.0 equiv) and $PdCl_2(MeCN)_2$ (87 mg, 0.34 mmol, 0.01 equiv.) were stirred in DMA (3.4 mL) and heated to 90 °C overnight. After cooling to room temperature the mixture was filtered through a pad of celite, which was rinsed three times with $Et_2O$. The filtrate was washed with $H_2O$ and the aqueous phase was extracted two times with $Et_2O$. The combined organic layers were washed with brine, dried over $Na_2SO_4$ and evaporated. The residue was purified by column chromatography (25% EA in PE) to afford the desired as a yellow solid (0.75 g, 3.0 mmol, **66**%). **Mp:** 125-127 °C,

**$^1$H-NMR (400 MHz, $CDCl_3$): δ =** 0.97 (t, *J* = 7.3 Hz, 3H), 1.60 - 1.75 (m, 2H), 1.80 - 1.91 (m, 4H), 2.62 - 2.70 (m, 2H), 2.86 - 2.95 (m, 2H), 3.13-3.23 (m, 1H), 3.86 (s, 3H), 8.85 (s, 1H) **$^{13}$C-NMR (101 MHz, $CDCl_3$): δ =** 14.0, 22.0, 22.0, 23.4, 25.3, 29.7, 42.2, 51.5, 77.5, 116.5, 123.3, 134.4, 142.8, 161.9, 199.8 **HRMS (pos. ESI): [M + H]$^+$** calc. for $C_{14}H_{20}NO_3$: 250.1438; found: 250.1438.

For methyl 3-butyl-4-oxo-2,4,5,6,7,8-hexahydrocyclohepta[c]pyrrole-1-carboxylate 1-bromobutane (0.97 mL, 1.2 g, 9.1 mmol, 2.0 equiv) was used instead of 1-bromopropane. The desired product was obtained as a yellow solid (0.78 g, 3.0 mmol, **59**%). **Mp:** 123-125 °C,

**$^1$H-NMR (500 MHz, $CDCl_3$):** δ = 0.93 (t, *J* = 7.3 Hz, 3H), 1.38 (dq, *J* = 14.7, 7.3 Hz, 2H), 1.55 - 1.66 (m, 2H), 1.79 - 1.88 (m, 4H), 2.61 - 2.69 (m, 2H), 2.88 - 2.95 (m, 2H), 3.14 - 3.21 (m, 2H), 3.86 (s, 3H), 8.97 (s, 1H). $^{13}$C-**NMR (126 MHz, $CDCl_3$):** δ = 14.0, 21.9, 22.7, 23.3, 25.3, 27.5, 30.9, 42.1, 51.5, 116.5, 123.1, 134.5, 143.1, 162.0, 199.9. **HRMS (pos. ESI): [M + H]$^+$** calc. for $C_{15}H_{22}NO_3$: 264.1594; found: 264.1596.

**4-Oxo-3-propyl-2,4,5,6,7,8-hexahydrocyclohepta[c]pyrrole-1-carboxylic acid and (step v).**

LiOH•$H_2O$ (1.3 g, 30 mmol, 10 equiv) was added to a solution of methyl 4-oxo-3-propyl-2,4,5,6,7,8-hexahydrocyclohepta[*c*]pyrrole-1-carboxylate (0.75 g, 3.0 mmol, 1.0 equiv) in a mixture of THF/$H_2O$/MeOH (30 mL, 1:1:1). The reaction mixture was stirred at rt overnight. The organic solvents were evaporated and the aqueous phase was acidified with conc. HCl, extracted three times with EA, dried over $Na_2SO_4$ and concentrated *in vacuo* to obtain the desired product as a colourless solid (0.69 g, 2.9 mmol, **97**%). **Mp:** 182-187 °C,

**$^1$H-NMR (500 MHz, Acetone-$d_6$):** $\delta$ **=** 0.90 (t, *J* = 7.4 Hz, 3H), 1.59 - 1.69 (m, 2H), 1.77 - 1.85 (m, 4H), 2.54 - 2.64 (m, 2H), 2.85 - 2.96 (m, 2H), 3.18 - 3.29 (m, 2H), 10.77 (s, 1H). **$^{13}$C-NMR (126 MHz, Acetone-$d_6$):** $\delta$ **=** 14.1, 22.5, 23.4, 26.0, 29.5, 42.4, 117.3, 123.5, 134.5, 143.4, 162.4, 199.0. **HRMS (neg. APCI): [M - H]$^-$** calc. for $C_{13}H_{16}NO_3$. 234.1136; found: 234.1130

**4-Oxo-3-butyl-2,4,5,6,7,8-hexahydrocyclohepta[*c*]pyrrole-1-carboxylic acid (step v)**

LiOH•$H_2O$ (1.2 g, 30 mmol, 10 equiv) was added to a solution of methyl 4-oxo-3-butyl-2,4,5,6,7,8-hexahydrocyclohepta[*c*]pyrrole-1-carboxylate (0.78 g, 3.0 mmol, 1.0 equiv) in a mixture of THF/$H_2O$/MeOH (30 mL, 1:1:1). The reaction mixture was stirred at rt overnight. The organic solvents were evaporated and the aqueous phase was acidified with conc. HCl, extracted three times with EA, dried over $Na_2SO_4$ and concentrated *in vacuo* to obtain the desired product as a colourless solid (0.20 g, 0.79 mmol, 27%). Mp: 134-139 °C.

**$^1$H-NMR (500 MHz, Acetone-$d_6$):** $\delta$ **=** 0.90 (t, J = 7.4 Hz, 3H), 1.28 - 1.39 (m, 2H), 1.55 - 1.64 (m, 2H), 1.76 - 1.85 (m, 4H), 2.55 - 2.62 (m, 2H), 2.90 - 2.99 (m, 2H), 3.20 - 3.27 (m, 2H), 10.79 (s, 1H). $^{13}$C-NMR **(126 MHz, Acetone-$d_6$):** $\delta$ = 14.1, 22.5, 23.2, 23.4, 26.0, 27.4, 32.4, 42.4, 117.5, 123.4, 134.4, 143.5, 162.8, 199.0. **HRMS (neg. APCI):** [M + H]$^+$ calc. for $C_{14}H_{18}NO_3$. 248.1292; found: 248.1291.

**1-Bromo-3-propyl-5,6,7,8-tetrahydrocyclohepta[c]pyrrol-4(2*H*)-one.**

Pyridine (1.0 mL, 0.98 g, 13 mmol, 5.0 equiv) and $PyHBr_3$ (1.2 g, 3.6 mmol, 1.5 equiv) were added to a solution of 4-oxo-3-propyl-2,4,5,6,7,8-hexahydrocyclohepta[c]pyrrole-1-carboxylic acid (0.57 g, 2.4 mmol, 1.0 equiv) in DCM (24 mL) at 0 °C. The reaction mixture was stirred at rt for 4.5 h. Aqueous HCl (0.5 M) was added and the phases were separated. The organic phase was dried over $Na_2SO_4$ and concentrated *in vacuo.* The residue was purified via column chromatography (5-9% EA in pentane) to obtain the desired product as a colourless solid which turns black and decomposes at rt (0.43 g, 1.6 mmol, **66**%). The product was used without further characterisation due to it's instability. **HRMS (pos. ESI): [M + H]$^+$** calc. for $C_{12}H_{17}NOBr$: 270.0488; found: 270.0489.

**1-Bromo-3-butyl-5,6,7,8-tetrahydrocyclohepta[*c*]pyrrol-4(2*H*)-one**

Pyridine (0.28 mL, 0.27 g, 3.4 mmol, 5.0 equiv) and $PyHBr_3$ (0.33 g, 1.0 mmol, 1.5 equiv) were added to a solution of 4-oxo-3-butyl-2,4,5,6,7,8-hexahydrocyclohepta[*c*]pyrrole-1-carboxylic acid (0.17 g, 0.68 mmol, 1.0 equiv) in DCM (7.0 mL) at 0 °C. The reaction mixture was stirred at rt for 4.5 h. Aqueous HCl (0.5 M) was added and the phases were separated. The organic phase was dried over $Na_2SO_4$ and concentrated *in vacuo.* The residue was purified via column chromatography (5-6% EA in pentane) to obtain the desired product as a colourless solid which turns black and decomposes at rt (79 mg, 0.28 mmol, **41**%). The product was used without further characterisation due to it's instability. **HRMS (pos. ESI): [M + H]$^+$** calc. for $C_{13}H_{19}NOBr$: 284.0645; found: 284.0643.

**1-(2-Chloro-5-nitrophenyl)-3-propyl-5,6,7,8-tetrahydrocyclohepta[c]pyrrol-4(2*H*)-one**

To 1-bromo-3-propyl-5,6,7,8-tetrahydrocyclohepta[*c*]pyrrol-4(2*H*)-one (100 mg, 0.37 mmol, 1.0 equiv.), 2-(2-Chloro-5-nitrophenyl)-4,4,5,5-tetramethyl-1,3,2-dioxaborolane (0.16 g, 0.56 mmol, 1.5 equiv.), 1,3,5,7-tetramethyl-6-phenyl-2,4,8-trioxa-6-phosphaadamantane (22 mg, 74 $\mu$mol, 0.02 equiv.), $Pd_2dba_3$ (17 mg, 19 $\mu$mol, 0.05 equiv.), $K_3PO_4$ (0.24 g, 1.1 mmol, 3.0 equiv.) in degassed dioxane/$H_2O$ (1.9 mL) was stirred 2.5 h at 70 °C, then filtered over celite and evaporated. The residue was purified by column chromatography (3-85% EA in PE) to afford the desired product (25 mg, 73 $\mu$mol, **20**%) as a light yellow solid. **Mp:** 68-70 °C.

**$^1$H-NMR (500 MHz, $CDCl_3$):** $\delta$ = 0.98 (t, *J* = 7.4, 7.4 Hz, 3H), 1.61 - 1.75 (m, 2H), 1.77 - 1.93 (m, 4H), 2.64 - 2.72 (m, 4H), 2.89 - 2.99 (m, 2H), 7.63 (d, *J* = 8.8 Hz, 1H), 8.10 (dd, *J* = 8.8, 2.7 Hz, 1H), 8.19 (d, *J* = 2.7 Hz, 1H), 8.65 (s, 1H). **$^{13}$C-NMR (126 MHz, $CDCl_3$):** $\delta$ = 14.0, 22.1, 22.4, 23.8, 25.5, 29.6, 42.0, 121.1, 121.4, 123.2, 125.6, 127.0, 131.3, 132.7, 140.3, 140.8, 146.6, 200.0. **HRMS (pos. ESI): [M + H]$^+$** calc. for $C_{18}H_{20}N_2O_3Cl$: 347.1157; found: 347.1160.

**1-(2-Chloro-5-nitrophenyl)-3-butyl-5,6,7,8-tetrahydrocyclohepta[*c*]pyrrol-4(2*H*)-one.**

A mixture of 1-bromo-3-butyl-5,6,7,8-tetrahydrocyclohepta[*c*]pyrrol-4(2*H*)-one (0.8 g, 0.28 mmol, 1.0 equiv.), 2-(2-Chloro-5-nitrophenyl)-4,4,5,5-tetramethyl-1,3,2-dioxaborolane (0.12 g, 0.42 mmol, 1.5 equiv.), 1,3,5,7-tetramethyl-6-phenyl-2,4,8-trioxa-6-phosphaadamantane (16 mg, 56 μmol, 0.02 equiv.), $Pd_2dba_3$ (13 mg, 14 μmol, 0.05 equiv.), $K_3PO_4$ (0.18 g, 0.85 mmol, 3.0 equiv.) in degassed dioxane/$H_2O$ (1.4 mL) was stirred 2.5 h at 70 °C, then filtered over celite and evaporated. The residue was purified by reversed phase column chromatography (MeCN/$H_2O$) to afford the desired product (12 mg, 33 μmol, **12**%) as a yellow oil. **$^1$H-NMR (500 MHz, $CDCl_3$): δ =** 0.94 (t, *J* = 7.3 Hz, 3H), 1.41 (dq, *J* = 14.7, 7.4 Hz, 2H), 1.61 - 1.69 (m, 2H), 1.81 - 1.95 (m, 4H), 2.69 (ddd, *J* = 7.6, 4.4, 1.8 Hz, 4H), 2.94 - 3.03 (m, 2H), 7.64 (d, *J* = 8.8 Hz, 1H), 8.11 (dd, *J* = 8.8, 2.7 Hz, 1H), 8.19 (d, *J* = 2.7 Hz, 1H), 8.44 (s, 1H). **$^{13}$C-NMR (126 MHz, $CDCl_3$): δ =** 14.1, 22.1, 22.7, 23.8, 25.6, 27.4, 31.2, 42.1, 121.0, 121.5, 123.2, 125.7, 126.9, 131.3, 132.7, 140.2, 140.9, 146.6, 199.9. **HRMS (neg. ESI): [M - H]-** calc. for $C_{19}H_{20}N_2O_3Cl$: 359.1168; found: 359.1165.

**1-(5-Amino-2-chlorophenyl)-3-propyl-5,6,7,8-tetrahydrocyclohepta[*c*]pyrrol-4(2*H*)-one (Compound 26).**

To a solution of 1-(2-chloro-5-nitrophenyl)-3-propyl-5,6,7,8-tetrahydrocyclohepta[*c*]pyrrol-4(2*H*)-one (25 mg, 72 μmol, 1.0 equiv.) and $NH_4Cl$ (7.7 mg, 0.14 mmol, 2.0 equiv.) in EtOH/$H_2O$ (4:1, 1.4 mL) Fe powder (14 mg, 0.25 mmol, 3.5 equiv.) was added. The mixture was stirred under reflux for 4 h, afterwards filtered first over cotton, then celite. EtOH was evaporated, water was added and the aqueous phase was three times extracted with EA. The combined organic phases were washed with brine, dried over $Na_2SO_4$ and evaporated. The residue was purified by reversed phase column chromatography (MeCN/$H_2O$) to afford the desired product as a yellow oil (15 mg, 47 μmol, **66**%).

**$^1$H-NMR (500 MHz, $CDCl_3$):** δ = 0.98 (t, *J* = 7.3 Hz, 4H), 1.63 - 1.73 (m, 2H), 1.76 - 1.92 (m, 4H), 2.64 - 2.68 (m, 2H), 2.68 - 2.72 (m, 2H), 2.90-2.97 (m, 2H), 6.58 - 6.63 (m, 2H), 7.18 - 7.23 (m, 1H), 8.37 (s, 1H). $^{13}$C-**NMR (126 MHz, $CDCl_3$):** δ = 14.1, 22.3, 22.3, 23.7, 25.7, 29.7, 42.1, 115.8, 118.4, 121.0, 122.5, 123.5, 123.7, 130.8, 131.6, 139.3, 145.2, 200.2. **HRMS (pos. ESI): $[M + H]^+$** calc. for $C_{18}H_{22}N_2OCl$: 317.1415; found: 317.1422.

**1-(5-Amino-2-chlorophenyl)-3-butyl-5,6,7,8-tetrahydrocyclohepta[*c*]pyrrol-4(2*H*)-one (Compound 27).** To a solution of 1-(2-chloro-5-nitrophenyl)-3-butyl-5,6,7,8-tetrahydrocyclohepta[c]pyrrol-4(2*H*)-one (12 mg, 33 μmol, 1.0 equiv.) and $NH_4Cl$ (3.5 mg, 66 μmol, 2.0 equiv.) in EtOH/$H_2O$ (4:1, 0.67 mL) Fe powder (6.5 mg, 0.12 mmol, 3.5 equiv.) was added. The mixture was stirred under reflux for 4 h, afterwards filtered first over cotton, then celite. EtOH was evaporated, water was added and the aqueous phase was three times extracted with EA. The combined organic phases were washed with brine, dried over $Na_2SO_4$ and evaporated. The residue was purified by reversed phase column chromatography (MeCN/$H_2O$) to afford the desired product as a yellow oil (6.6 mg, 20 μmol, **60**%). **$^1$H-NMR (500 MHz, $CDCl_3$):** δ **=** 0.93 (t, *J* = 7.3 Hz, 3H), 1.40 (h, *J* = 7.4 Hz, 2H), 1.58 - 1.69 (m, 2H), 1.76 - 1.92 (m, 4H), 2.63 - 2.74 (m, 4H), 2.93 - 3.00 (m, 2H), 6.56 - 6.68 (m, 2H), 7.21 (d, *J* = 8.3 Hz, 1H), 8.30 (s, 1H). $^{13}$C-**NMR (126 MHz, $CDCl_3$):** δ **=** 14.1, 22.3, 22.7, 23.8, 25.7, 27.5, 31.2, 42.1, 115.8, 118.5, 120.9, 122.6, 123.5, 123.7, 130.9, 131.6, 139.6, 145.1, 200.2. **HRMS (pos. ESI): $[M + H]^+$** calc. for $C_{19}H_{24}N_2OCl$: 331.1572; found: 331.1576.

**Protein preparation, crystallization and structure determination**

BRD4-BD1 was expressed and purified as described previously (Filippakopoulos et al, (2012), Cell 149(1): 241-231) with the exception of the final buffer for crystallization and ITC analysis (20 mM Hepes/NaOH pH 7.5, 150 mM NaCl). Crystals were grown in the presence of 3.5 M Na-Formate (pH 7.5) at a protein concentration of 10 mg/ml and a ligand concentration of 2 mM added directly to the protein prior to crystallization from a 100 mM stock solution in DMSO. Data were collected at 100K using either a Rigaku HF-007 rotating anode X-ray generator equipped with VariMaxHF optics and a Saturn944 CCD detector or a mar345 image plate respectively at λ = 1.54179 Å or at the PXI beamline at the Swiss Light Source at λ = 1.000 Å with a Pilatus detector. Data processing and reduction was done with iMOSFLM (Leslie AGW PH (2007), Evolving Methods for Macromolecular Crystallography 245, 41.51), POINTLESS, and SCALA ( Kabsch W (2010) Xds. Acta crystallographica. Section D, Biological crystallography 66(Pt 2):125-132; Bruker (2008) SADABS, SAINT and XPREP (Bruker AXS Inc., Madison, Wisconsin, USA); Collaborative Computational Project N (1994) The CCP4 suite: programs for protein crystallography, Acta crystallographica, Section D, Biological crystallography 50(Pt 5):760-763), or with XDS, XSCALE, XDSCONV (Evans P (2006), Scaling and assessment of data quality, Acta crystallographica, Section D, Biological crystallography 62 (Pt 1):72-82), and XPREP(Evans PR (2011), An introduction to data reduction: space-group determination, scaling and intensity statistics, Acta crystallographica, Section D, Biological crystallography 67(Pt 4):282-292).

BRD4•ligand complexes crystallized in space group $P2_12_12_1$. The structures were solved by molecular replacement with PHASER (McCoy AJ, et al. (2007), Phaser crystallographic software, Journal of applied crystallography40(Pt 4):658-674) with apoBRD4 as search model (internal data) yielding one molecule per asymmetric unit. Compounds were modelled into 2Fo-Fc electron density maps using AFITT-CL (version 2.1.0, OpenEye Scientific Software, Inc., Santa Fe, NM, USA.) Model building and real space refinement was done with COOT (Murshudov GN, Vagin AA, & Dodson EJ (1997), Refinement of macromolecular structures by the maximum-likelihood method, Acta crystallographica. Section D, Biological crystallography 53(Pt 3):240-255) reciprocal space refinement against the calculated data was done with Refmac5, as implemented in the CCP4 suite (Murshudov GN, et al. (2011), REFMAC5 for the refinement of macromolecular crystal structures, Acta crystallographica. Section D, Biological crystallography 67(Pt 4):355-367; Vaguine AA, Richelle J, & Wodak SJ (1999), SFCHECK: a unified set of procedures for evaluating the quality of macromolecular structure-factor data and their agreement with the atomic model, Acta crystallographica, Section D, Biological crystallography 55(Pt 1):191-205). Final structure validation was done with procheck/sfcheck (Wlodek S, Skillman AG, & Nicholls A (2006), Automated ligand placement and refinement with a combined force field and shape potential, Acta crystallographica, Section D, Biological crystallography 62(Pt 7):741-749).

**Isothermal titration calorimetry**

ITC experiments for the determination of the dissociation constant $K_d$ were done with a Microcal VP-ITC microcalorimeter (GE Healthcare) at 25 °C using ligand concentrations between 10 and 50 μM in the sample cell and BRD4 concentrations between 120 and 600 μM in the injection syringe. Data were obtained in discrete titration experiments with an injection volume of 12 μl per injection. Subsequently, the heats per injection were calculated as integrals and after normalization against the molar concentrations plotted against the molar ratio as implemented in Microcal Origin. Finally, data were fitted according to a single set of sites binding model with

$$(1) \qquad Q = nM_t \times \Delta HV_0 \times \frac{1}{2} \times \left(1 + \frac{X_t}{nM_t} + \frac{1}{nKM_t} - \sqrt{\left(1 + \frac{X_t}{nM_t} + \frac{1}{nKM_t}\right)^2 - \frac{4X_t}{nM_t}}\right)$$

as function to calculate the overall sum of heat of the titration and with

$$(2) \qquad \Delta Q(i) = Q(i) + \frac{dV_i}{V_0} \times \left(\frac{Q(i)+Q(i-1)}{2}\right) - Q(i-1)$$

(According to Microcal's manual "ITC Data Analysis in Origin" (September 1998)) as function describing the sum of heat of each individual injection. A correction term was included to compensate for displacement of volume of the sample cell in the course of subsequent injections according to the manufacturer's manual "ITC Data Analysis in Origin" (Microcal).

The dissociation constant $K_d$ is commonly used to describe the affinity between a ligand L and a protein, i.e. how tightly a ligand binds to a particular protein. The dissociation constant has molar units which correspond to the concentration of ligand at which the binding site on a particular protein is half occupied, i.e. the concentration of the ligand at which the concentration of protein with ligand bound equals the concentration of protein with no ligand bound. The smaller the dissociation constant, the higher the affinity between ligand and protein.

Table 1 shows the dissociation constants $K_D$ for two bromodomains (BRD4(1) and BRPF1B) as well as the concentration of the respective compound required to reduce the population of a culture of HL-60 cells by 50% ($GI_{50}$)

Table 1 (n.d. = not determined)

| Cpd. of formula | $K_D$ BRD4(1) (nM) | $K_D$ BRPF1B (nM) | $GI50_{HL60}$ (nM) |
|---|---|---|---|
| 1" | 6410 | 5080 | n.d. |
| 8 | 4562 | 1050 | n.d. |
| 9 | 8281 | 2050 | n.d. |
| 11 | 143.9 | n.d. | 1360 |
| 12 | 176.7 | n.d. | 570 |
| 13 | 77.8 | n.d. | 170 |
| 14 | 259.6 | n.d. | 2080 |
| 15 | 47.3 | n.d. | n.d. |
| 16 | 417.2 | n.d. | n.d. |
| 18 | 72.2 | n.d. | n.d. |
| 19 | n.d. | 910 | n.d. |

(continued)

| Cpd. of formula | $K_D$ BRD4(1) (nM) | $K_D$ BRPF1B (nM) | $GI50_{HL60}$ (nM) |
|---|---|---|---|
| 20 | n.d. | 567 | n.d. |
| 21 | n.d. | 1990 | n.d. |
| 22 | n.d. | 2310 | n.d. |
| 23 | n.d. | 166 | n.d. |
| 24 | n.d. | 639 | n.d. |
| 25 | n.d. | 713 | n.d. |
| 26 | 6900 | 7000 | n.d. |
| 27 | > 10000 | > 10000 | |

**Bromodomain profiling**

Bromodomain profiling was carried out on the basis of BROMOscan™. This platform accounted for the indirect determination of the dissociation constants between 19 bromodomains and the compound of formula (13), by binding competition against a reference immobilized ligand.

The overall structure of the complex of the compound of formula (13) and BRD4-BD1 revealed the already well-characterized bromodomain fold with a bundle of four $\alpha$-helices, interconnected by three loops of different length. The termini of the helices bundle are flanked by elongational loops, which tightly pack against the protein core producing a compact and rather rigid structure.

The compound of formula (13) is bound in a pocket located at the end of the longitudinal axis running through the helix bundle which points towards the N-terminus. Consequently, it occupies the same pocket as the native $K_{ac}$ substrate.

Moreover, it mimics the $K_{ac}$ interaction with BRD4-BD1 by positioning the 4-acyl substitution in the pyrrole ring toward the highly conserved Asn140, thus engaging in hydrogen bond interactions with Asn140 and the equally conserved water molecule that bridges to the conserved Tyr97. The pyrrole ring is located deep in the recognition pocket, and complements the hydrophobic pocket defined by the four conserved waters with a 5-methyl substitution. The surface complementarity between the ligand and the recognition pocket is further achieved by the 3-ethyl substitution in the pyrrole ring. The presence of the heteroatom in the core of the compound of formula (I") allows for a key hydrogen-bond donor interaction with the proline's backbone in position 82. Such interaction has not been described previously, and may have an important role in fixating the compound in the recognition site.

Apart from this major determinant of ligand recognition by BRD4-BD1, the compound of formula (13) also explores another patch of interactions.

The phenylsulphonamide moiety is placed along the ZA channel, such that a T-shaped CH-$\pi$ interaction with Trp81 is established, whereby a perfect orthogonal orientation of both aromatic systems is created withTrp81 directly pointing towards the centre of the phenyl moiety of the compound of formula (13). Such interactions have already been reported in other drug-protein interactions. In addition, Leu92 serves as lid from the opposing side and together with Trp81 it forms a special configuration which will be referred to hereinafter as WL trap.

Compounds that bind the bromodomain prevent its binding to the immobilized ligand thus reducing the amount of protein captured. Conversely, molecules that do not bind the bromodomain have no effect on the amount of protein captured. Hits are identified by measuring the amount of bromodomain captured in test versus control samples.

**Protocol description**

**Bromodomain assays for Kd determination:** T7 phage strains displaying bromodomains were grown in parallel in 24-well blocks in an E. coli host derived from the BL21 strain. E. coli were grown to log-phase and infected with T7 phage from a frozen stock (multiplicity of infection = 0.4) and incubated with shaking at 32°C until lysis (90-150 minutes). The lysates were centrifuged (5,000 x g) and filtered (0.2$\mu$m) to remove cell debris. Streptavidin-coated magnetic beads were treated with biotinylated small molecule or acetylated peptide ligands for 30 minutes at room temperature to generate affinity resins for bromodomain assays. The liganded beads were blocked with excess biotin and washed with blocking buffer (SeaBlock (Pierce), 1 % BSA, 0.05 % Tween 20, 1 mM DTT) to remove unbound ligand and to reduce non-specific phage binding. Binding reactions were assembled by combining bromodomains, liganded affinity beads, and test compounds in 1x binding buffer (17% SeaBlock, 0.33x PBS, 0.04% Tween 20, 0.02% BSA,

0.004% Sodium azide, 7.4 mM DTT). Test compounds were prepared as 1000X stocks in 100% DMSO. Kds were determined using an 11-point 3-fold compound dilution series with one DMSO control point. All compounds for Kd measurements are distributed by acoustic transfer (non-contact dispensing) in 100% DMSO. The compounds were then diluted directly into the assays such that the final concentration of DMSO was 0.09%. All reactions performed in polypropylene 384- well plates. Each was a final volume of 0.02 ml. The assay plates were incubated at room temperature with shaking for 1 hour and the affinity beads were washed with wash buffer (1x PBS, 0.05% Tween 20). The beads were then re-suspended in elution buffer (1x PBS, 0.05% Tween 20, 2 μM non-biotinylated affinity ligand) and incubated at room temperature with shaking for 30 minutes. The bromodomain concentration in the eluates was measured by qPCR.

**Compound Handling for Kd determination:** An 11-point 3-fold serial dilution of each test compound was prepared in 100% DMSO at 1000x final test concentration. All compounds for Kd measurements are distributed by acoustic transfer (non-contact dispensing) in 100% DMSO. The compounds were then diluted directly into the assays such that the final concentration of DMSO was 0.09%. Most Kds were determined using a compound top concentration = 10,000 nM. If the initial Kd determined was < 0.169 nM (the lowest concentration tested), the measurement was repeated with a serial dilution starting at a lower top concentration.

Binding Constants (Kds): Binding constants (Kds) were calculated with a standard dose-response curve using the Hill equation:

$$\text{Response} = \frac{\text{Background} + \text{Signal} - \text{Background}}{1 + (\text{Kd}^{\text{Hill Slope}} / \text{Dose}^{\text{Hill Slope}})}$$

The Hill Slope was set to -1. Curves were fitted using a non-linear least square fit with the Levenberg-Marquardt algorithm.

The results are given in Table 2 and Table 3.

Table 2

| | Compound | | | | | | |
|---|---|---|---|---|---|---|---|
| **Bromodomain - (Gene symbol)** | **23** | **11** | **12** | **13** | **14** | **15** | **16** |
| | Kd (nM) | Kd (nM) | Kd (nM) | Kd (nM) | Kd (nM) | Kd (nM) | Kd (nM) |
| ATAD2A | >10000 | n.d. | n.d. | n.d. | n.d. | n.d. | n.d. |
| ATAD2B | >10000 | n.d. | n.d. | n.d. | n.d. | n.d. | n.d. |
| BAZ2A | 1900 | n.d. | n.d. | n.d. | n.d. | n.d. | n.d. |
| BAZ2B | 1800 | n.d. | n.d. | n.d. | n.d. | n.d. | n.d. |
| BRD1 | 3100 | n.d. | n.d. | n.d. | n.d. | n.d. | n.d. |
| BRD2(1) | 130 | n.d. | n.d. | 45 | n.d. | n.d. | n.d. |
| BRD2(1,2) | 84 | n.d. | n.d. | n.d. | n.d. | n.d. | n.d. |
| BRD2(2) | 200 | n.d. | n.d. | 290 | n.d. | n.d. | n.d. |
| BRD3(1) | 170 | n.d. | n.d. | 100 | n.d. | n.d. | n.d. |
| BRD3(1,2) | 100 | n.d. | n.d. | n.d. | n.d. | n.d. | n.d. |
| BRD3(2) | 330 | n.d. | n.d. | 140 | n.d. | n.d. | n.d. |
| BRD4(1) | 250 | 85 | 170 | 60 | 300 | 82 | 150 |
| BRD4(1,2) | 160 | n.d. | n.d. | n.d. | n.d. | n.d. | n.d. |
| BRD4(2) | 270 | n.d. | n.d. | 220 | n.d. | n.d. | n.d. |
| BRD4(short-iso.) | 180 | n.d. | n.d. | n.d. | n.d. | n.d. | n.d. |
| BRD7 | 430 | 250 | 310 | 85 | 160 | 440 | 5.5 |
| BRD8(1) | 2600 | n.d. | n.d. | n.d. | n.d. | n.d. | n.d. |
| BRD8(2) | >10000 | n.d. | n.d. | n.d. | n.d. | n.d. | n.d. |
| BRD9 | 67 | 160 | 530 | 67 | 68 | 300 | 4.3 |

(continued)

| | Compound | | | | | | |
|---|---|---|---|---|---|---|---|
| **Bromodomain - (Gene symbol)** | **23** | **11** | **12** | **13** | **14** | **15** | **16** |
| | Kd (nM) | Kd (nM) | Kd (nM) | Kd (nM) | Kd (nM) | Kd (nM) | Kd (nM) |
| BRDT(1) | 240 | n.d. | n.d. | 670 | n.d. | n.d. | n.d. |
| BRDT(1,2) | 420 | n.d. | n.d. | n.d. | n.d. | n.d. | n.d. |
| BRDT(2) | 620 | n.d. | n.d. | 1000 | n.d. | n.d. | n.d. |
| BRPF1 | 130 | 420 | 2500 | 520 | 320 | 1800 | 1300 |
| BRPF3 | 6000 | n.d. | n.d. | n.d. | n.d. | n.d. | n.d. |
| CECR2 | 970 | 1100 | 8100 | n.d. | 240 | 580 | 760 |
| CREBBP | 420 | n.d. | n.d. | n.d. | n.d. | n.d. | n.d. |
| EP300 | 1200 | 2100 | 9000 | n.d. | 1800 | 3500 | 990 |
| FALZ | 2600 | n.d. | n.d. | n.d. | n.d. | n.d. | n.d. |
| GCN5L2 | >10000 | n.d. | n.d. | n.d. | n.d. | n.d. | n.d. |
| PBRM1(2) | 4200 | n.d. | n.d. | n.d. | n.d. | n.d. | n.d. |
| PBRM1(5) | 3800 | n.d. | n.d. | n.d. | n.d. | n.d. | n.d. |
| PCAF | >10000 | n.d. | n.d. | n.d. | n.d. | n.d. | n.d. |
| SMARCA2 | >10000 | n.d. | n.d. | n.d. | n.d. | n.d. | n.d. |
| SMARCA4 | >10000 | n.d. | n.d. | n.d. | n.d. | n.d. | n.d. |
| TAF1(2) | 4100 | n.d. | n.d. | n.d. | n.d. | n.d. | n.d. |
| TAF1L(2) | 6800 | n.d. | n.d. | n.d. | n.d. | n.d. | n.d. |
| TRIM24 (Bromo.) | 1400 | n.d. | n.d. | n.d. | n.d. | n.d. | n.d. |
| TRIM24(PHD, Bromo.) | 5300 | n.d. | n.d. | n.d. | n.d. | n.d. | n.d. |
| TRIM33(PHD, Bromo.) | 7700 | n.d. | n.d. | n.d. | n.d. | n.d. | n.d. |
| WRD9(2) | >10000 | n.d. | n.d. | n.d. | n.d. | n.d. | n.d. |

**Table 3 (Results for compounds 23, 26 and 27)**

| **Bromodomain (Gene Symbol)** | **Compound** | | |
|---|---|---|---|
| | **23** | **26** | **27** |
| | Kd (nM) | Kd (nM) | Kd (nm) |
| BRD4(1) | 250 | 6900 | >10000 |
| BRD7 | 430 | 1100 | >10000 |
| BRD9 | 67 | 150 | 800 |
| BRDT(1) | 240 | 6100 | >10000 |
| BRPF1 | 130 | 7000 | >10000 |
| CECR2 | 970 | >10000 | >10000 |

The results show that the compounds of formula (26) and (27) have a higher selectivity for certain bromodomains evaluated compared to the compound of formula (23) which, in turn, shows a very good activity as inhibitor (as evidenced by low values for Kd) for all bromodomains evaluated in Table 3.

**Bromodomain assays for % Ctrl determination:** T7 phage strains displaying bromodomains were grown in

parallel in 24-well blocks in an E. coli host derived from the BL21 strain. E. coli were grown to log-phase and infected with T7 phage from a frozen stock (multiplicity of infection = 0.4) and incubated with shaking at 32°C until lysis (90-150 minutes). The lysates were centrifuged (5,000 x g) and filtered (0.2μm) to remove cell debris. Streptavidin-coated magnetic beads were treated with biotinylated small molecule or acetylated peptide ligands for 30 minutes at room temperature to generate affinity resins for bromodomain assays. The liganded beads were blocked with excess biotin and washed with blocking buffer (SeaBlock (Pierce), 1 % BSA, 0.05 % Tween 20, 1 mM DTT) to remove unbound ligand and to reduce non-specific phage binding. Binding reactions were assembled by combining bromodomains, liganded affinity beads, and test compounds in 1x binding buffer (16 % SeaBlock, 0.32x PBS, 0.02%BSA, 0.04 % Tween 20, 0.004% Sodium azide, 7.9 mM DTT). Test compounds were prepared as 1000X stocks in 100% DMSO and subsequently diluted 1:25 in monoethylene glycol (MEG). The compounds were then diluted directly into the assays such that the final concentrations of DMSO and MEG were 0.1% and 2.4%, respectively. All reactions were performed in polypropylene 384-well plates in a final volume of 0.02 ml. The assay plates were incubated at room temperature with shaking for 1 hour and the affinity beads were washed with wash buffer (1x PBS, 0.05% Tween 20). The beads were then re-suspended in elution buffer (1x PBS, 0.05% Tween 20, 2 μM non-biotinylated affinity ligand) and incubated at room temperature with shaking for 30 minutes. The bromodomain concentration in the eluates was measured by qPCR.

The compounds were screened at a concentration of 10 000 nM and results for primary screen binding are reported as % of control where lower numbers indicate a stronger binding

The %Ctrl values were determined as follows:

$$\% \text{ Ctrl} = [(\text{test compound signal-positive control signal})/(\text{negative control signal-positive control signal})] \times 100$$

where test compound was the compound of formula (13), negative control was DMSO and positive control was the control compound

Table 3 shows the results for a group of 32 bromodomains:

Table 3

| Target bromodomain | % Ctrl @10 000 nM |
|---|---|
| ATAD2A | 94 |
| ATAD2B | 97 |
| BAZ2A | 24 |
| BAZ2B | 6.8 |
| BRD1 | 26 |
| BRD2(1) | 0 |
| BRD2(2) | 0.1 |
| BRD3(1) | 0.05 |
| BRD3(2) | 0 |
| BRD4(1) | 0 |
| BRD4(2) | 0 |
| BRD7 | 0 |
| BRD9 | 0 |
| BRDT(1) | 0 |
| BRDT(2) | 0.65 |
| BRPF1 | 0.15 |
| BRPF3 | 35 |
| CECR2 | 1.3 |
| CREBBP | 4.7 |

(continued)

| Target bromodomain | % Ctrl @10 000 nM |
|---|---|
| EP300 | 1.4 |
| FALZ | 6.8 |
| GCN5L2 | 44 |
| PBRM1(2) | 75 |
| PBRM1(5) | 90 |
| PCAF | 20 |
| SMARCA2 | 83 |
| SMARCA4 | 71 |
| TAF1 (2) | 7.4 |
| TAF1 L(2) | 40 |
| TRIM24(PHD, Bromo) | 82 |
| TRIM33(PHD, Bromo) | 60 |
| WDR9(2) | 81 |

The results show that the compounds showed very good to average binding affinity to a variety of bromodomains (less than 35 % Ctrl).

The results indicate that the compounds in accordance with the present invention provide a promising starting point for the development of novel potent bromodomain inhibitors.

*Plasmodium falciparum* inhibitor treatment for 72 hours (one replication cycle)

*P. falciparum* cultures were treated for 72 hours with 100 μm of compounds (19) to (22), (24) and (25) or DMSO as control. The cultures were stained for DNA content with fluorescent dye Hoechst 33342 (20 μm) and for RNA content with thiazole orange (1 μm). The double negative population represented uninfected erythrocytes (devoid of DNA and low in RNA content) whereas the *P. falciparum* infected erythrocytes were gated as early ring, mid trophozoite and late schizont stages based on their DNA and RNA content. The degree of parasitemia was determined by flow cytometry after 72 hours of drug treatment and the compounds in accordance with the present invention yielded a lower degree of parasitemia than the control with the compound of formula (24) being particularly effective in this regard in each of the stages (early ring, mid trophozoite and schizont).

## Claims

**1.** A compound of formula (1) or formula (2)

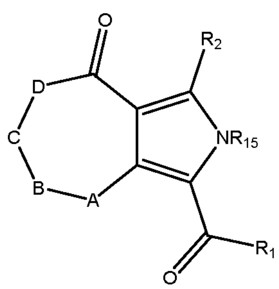

(1)

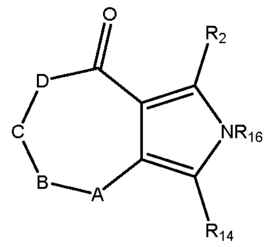

(2)

wherein

A, B, C and D, which may be the same or different, independent of one another represent $-C(R_7R_8)-$, $-C(R_9R_{10})-C(R_{11}R_{12})-$ , $-N(R_{13})-$, -O- or -S- with the proviso that the total number of ring atoms of the ring comprising ring members A, B, C and D is 7 to 9,
$R_1$ is selected from the group consisting of OH, $OR_3$, $NH_2$, $NHR_4$ and $NR_5R_6$ $R_2$ is a substituted or unsubstituted alkyl group with 1 to 8 carbon atoms or a substituted or unsubstituted alkoxy group with 1 to 8 carbon atoms in which one or more of the hydrogen atoms may be optionally replaced by halogen,
$R_3$ and $R_4$, independent of each other, represent an alkyl group with 1 to 6 carbon atoms or a substituted or unsubstituted alkylaryl, aryl or heteroaryl group,
$R_5$ and $R_6$, independent of each other, represent an alkyl group with 1 to 6 carbon atoms or a substituted or unsubstituted aryl or heteroaryl group or form together with the nitrogen atom to which they are attached, a six- or seven membered, substituted or unsubstituted heterocyclic ring,
$R_7$ to $R_{13}$, independent of each other, represent hydrogen, hydroxyl, substituted or unsubstituted alkyl groups with 1 to 4 carbon atoms, substituted or unsubstituted alkoxy groups with 1 to 4 carbon atoms and substituted or unsubstituted hydroxyalkyl groups with 1 to 4 carbon atoms or wherein two of $R_7$ to $R_{13}$ located at adjacent atoms, form together a chemical bond or a ring,
$R_{14}$ represents a substituted or unsubstituted aryl or heteroaryl group, and $R_{15}$ and $R_{16}$, independent of each other, are hydrogen or a $C_1$ to $C_4$ -alkyl group.

**2.** The compound of formula (1) or (2) in accordance with claim 1 wherein A, B, C and D are $-(CR_7R_8)-$.

**3.** The compound of formula (1) or (2) in accordance with claim 1 or 2 wherein $R_2$ is an alkyl group or a haloalkyl group with 1 to 4 carbon atoms

**4.** The compound of formula (1) in accordance with any of claims 1 to 3
wherein $R_1$ is a group $NHR_4$ wherein $R_4$ is as defined in claim 1.

**5.** The compound of formula (1) in accordance with claim 4 wherein $R_4$ is a substituted or unsubstituted aryl group, in particular a substituted or unsubstituted phenyl group.

**6.** The compound of formula (1) in accordance with claim 5 wherein $R_4$ is represented by formula (3)

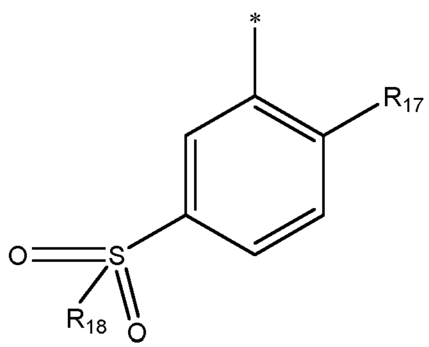

(3)

wherein * represents the site of attachment to the nitrogen atom,
$R_{17}$ is hydroxyl or an alkoxy group with 1 to 4 carbon atoms and $R_{18}$ is - $NR_{19}R_{20}$ wherein $R_{19}$ and $R_{20}$,

independent of each other, represent an alkyl group with 1 to 4 carbon atoms, a substituted or unsubstituted aryl ring or together with the nitrogen atom to which they are attached form a substituted or unsubstituted heterocyclic ring with 4 to 7 ring atoms.

**7.** The compound of formula (1) in accordance with any of claims 1 to 3 wherein $R_1$ is a group $-OR_3$ wherein $R_3$ is as defined in claim 1.

**8.** The compound of formula (1) in accordance with any of claims 1 to 4 represented by formula (1")

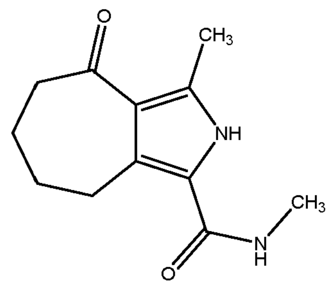

(1")

**9.** The compound of formula (2) in accordance with any of claims 1 to 3 wherein $R_{14}$ is a substituted or unsubstituted phenyl group or a substituted or unsubstituted oxadiazole group.

**10.** The compound of formula (2) in accordance with any of claims 1 to 3 or claim 9 wherein $R_2$ is an alkyl group with 1 to 4 carbon atoms.

**11.** The compound of formula (2) in accordance with any of claims 1 to 3, 9 or 10 wherein $R_{14}$ is a substituted or unsubstituted phenyl or oxadiazole group.

**12.** The compound of formula (1) in accordance with any of claims 1 to 6 represented by formula (4), formula (5), formula (6) or formula (7)

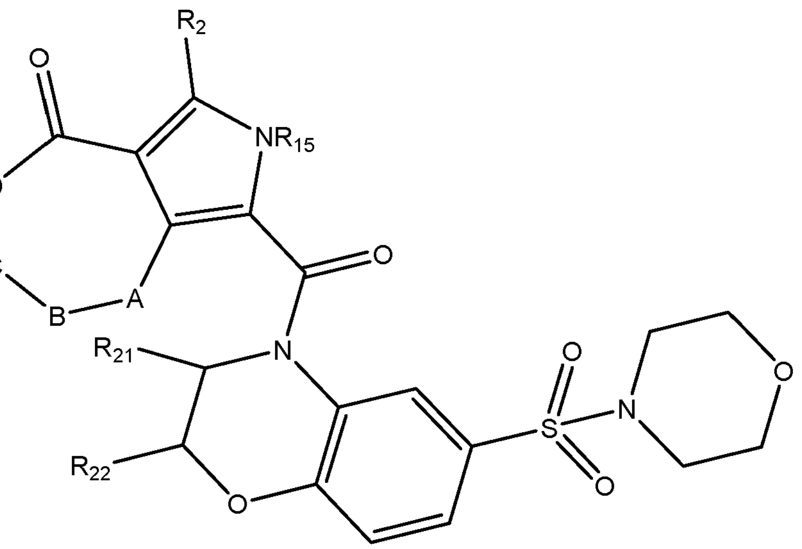

(4)

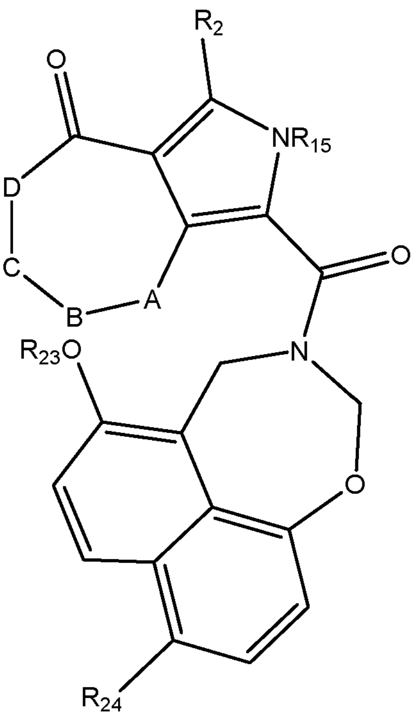

(5)

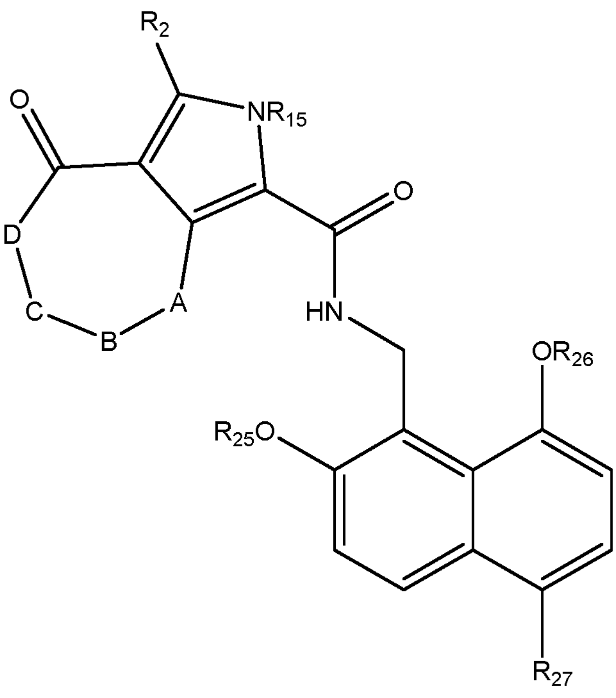

(6)

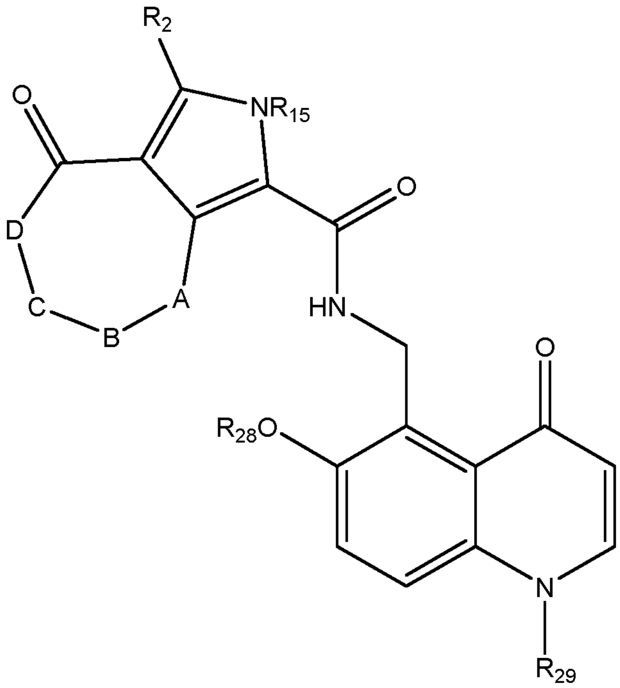

(7)

wherein A, B, C, D, $R_2$ and $R_{15}$ are as defined in claim 1,
$R_{21}$ and $R_{22}$, independent of each other, are hydrogen, $C_1$-$C_4$ alkyl or form together a chemical bond,
$R_{23}$, $R_{25}$ and $R_{28}$, independent of each other, are hydrogen or $C_1$ to $C_4$ alkyl,
$R_{24}$, $R_{27}$ and $R_{29}$, independent of each other, are $C_1$-$C_4$ alkyl or $C_2$-$C_4$-carboxyl, and
$R_{26}$ is hydrogen or $C_1$ to $C_4$ alkyl.

**13.** The compound of formula (1) or (2) in accordance with any of claims 1 to 12 for use in the treatment of diseases or conditions for which a bromodomain inhibitor is indicated.

**14.** A pharmaceutical composition comprising at least one compound of formula (1) or (2) as defined in any of claims 1 to 12 or a pharmaceutically acceptable salt thereof and one or more pharmaceutically acceptable carriers, diluents or excipients.

**15.** A combination pharmaceutical product comprising at least one compound of formula (1) or (2) as defined in any of claims 1 to 12 or a pharmaceutically acceptable salt thereof and one or more pharmaceutically acceptable carriers, diluents or excipients together with one or more other pharmaceutically active ingredients.

## Patentansprüche

**1.** Verbindung der Formel (1) oder Formel (2)

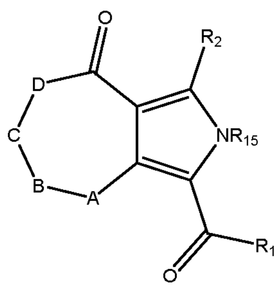

(1)

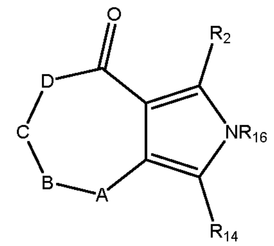

(2)

wobei

A, B, C and D, welche gleich oder unterschiedlich sein können, unabhängig voneinander $-C(R_7R_8)-$, $-C(R_9R_{10})-C(R_{11}R_{12})-$ , $-N(R_{13})-$, -O- or -S-darstellen, mit der Maßgabe dass die Gesamtzahl der Ringatome des Rings, der die Ringelemente A, B, C und D enthält, 7 bis 9 beträgt,
$R_1$ ausgewählt ist aus der Gruppe bestehend aus OH, $OR_3$, $NH_2$, $NHR_4$ und $NR_5R_6$
$R_2$ eine substituierte oder unsubstituierte Alkylgruppe mit 1 bis 8 C-Atomen oder eine substituierte oder unsubstituierte Alkoxygruppe mit 1 bis 8 C-Atomen, in denen ein oder mehrere Wasserstoffatome durch Halogenatome ersetzt sein können, ist
$R_3$ and $R_4$, unabhängig voneinander, eine Alkylgruppe mit 1 bis 6 C-Atomen oder eine substituierte oder unsubstituierte Alkylaryl-, Aryl oder Heteroarylgruppe sind,
$R_5$ and $R_6$, unabhängig voneinander, eine Alkylgruppe mit 1 bis 6 C-Atomen oder eine substituierte oder unsubstituierte Aryl- oder Heteroarylgruppe sind oder zusammen mit dem Stickstoffatom, an das sie gebunden sind, einen sechs- oder siebengliedrigen substituierten oder unsubstituierten heterocyclischen Ring bilden,
$R_7$ to $R_{13}$, unabhängig voneinander, Wasserstoff, Hydroxyl, substituierte oder unsubstituierte Alkylgruppen mit 1 bis 4 Kohlenstoffatomen, substituierte oder unsubstituierte Alkoxygruppen mit 1 bis 4 C-Atomen oder substituierte oder unsubstituierte Hydroxyalkylgruppen mit 1 bis 4 Kohlenstoffatomen darstellen oder zwei der Substituenten $R_7$ bis $R_{13}$ die an benachbarten Atomen angeordnet sind, zusammen eine chemische Bindung oder einen Ring bilden,
$R_{14}$ eine substituierte oder unsubstituierte Aryl- oder Heteroarylgruppe ist, und
$R_{15}$ and $R_{16}$, unabhängig voneinander, Wasserstoff oder eine $C_1$ bis $C_4$-Alkylgruppe sind.

2. Verbindung der Formel (1) oder (2) gemäß Anspruch 1 worin A, B, C und D $-(CR_7R_8)-$ sind

3. Verbindung der Formel (1) oder (2) gemäß Anspruch 1 oder 2 wobei $R_2$ eine Alkylgruppe oder eine Haloalkylgruppe mit 1-4 C-Atomen ist.

4. Verbindung der Formel (1) gemäß einem der Ansprüche 1 bis 3 wobei $R_1$ eine Gruppe $NHR_4$ ist und $R_4$ wie in Anspruch 1 definiert ist.

5. Verbindung der Formel (1) gemäß Anspruch 4 wobei $R_4$ eine substituierte oder unsubstituierte Arylgruppe, insbesondere eine substituierte oder unsubstituierte Phenylgruppe, ist.

6. Verbindung der Formel (1) gemäß Anspruch 5 wobei $R_4$ durch Formel (3) repräsentiert wird

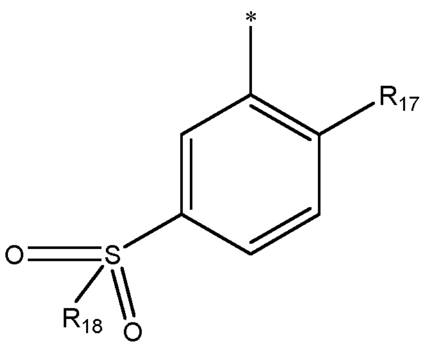

(3)

wobei * den Ort der Bindung an das Stickstoffatom darstellt,
$R_{17}$ eine Hydroxyl- oder Alkoxygruppe mit 1 bis 4 Kohlenstoffatomen darstellt und $R_{18}$ $-NR_{19}R_{20}$ ist, worin $R_{19}$ and $R_{20}$, unabhängig voneinander, eine Alkylgruppe mit 1 bis 4 Kohlenstoffatomen oder ein unsubstituierter oder unsubstituierter Arylring sind oder zusammen mit dem Stickstoffatom, an das sie gebunden sind, einen substituierten oder unsubstituierten heterocyclischen Ring mit 4 bis 7 Ringatomen bilden.

**7.** Verbindung der Formel (1) gemäß einem der Ansprüche 1 bis 3 wobei $R_1$ eine Grupper $-OR_3$ ist, wobei $R_3$ wie in Anspruch 1 definiert ist.

**8.** Verbindung der Formel (1) gemäß einem der Ansprüche 1 bis 4 repräsentiert durch Formel (1")

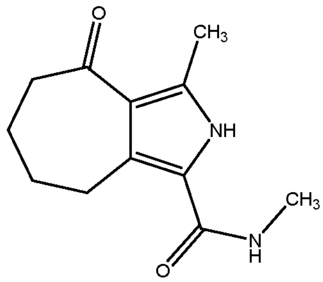

(1")

**9.** Verbindung der Formel (2) gemäß einem der Ansprüche 1 bis 3 wobei $R_{14}$ eine substituierte oder unsubstituierte Phenylgruppe oder eine substituierte oder unsubstituierte Oxadiazolgruppe ist.

**10.** Verbindung der Formel (2) gemäß einem der Ansprüche 1 bis 3 oder 9 wobei $R_2$ eine Alkylgruppe mit 1 bis 4 Kohlenstoffatomen ist.

**11.** Verbindung der Formel (2) gemäß einem der Ansprüche 1 bis 3, 9 oder 10 wobei $R_{14}$ eine substituierte oder unsubstituierte Phenyl- oder Oxadiazolgruppe ist.

**12.** Verbindung der Formel (1) gemäß einem der Ansprüche 1 bis 6 repräsentiert durch Formel (4), Formel (5), Formel (6) oder Formel (7)

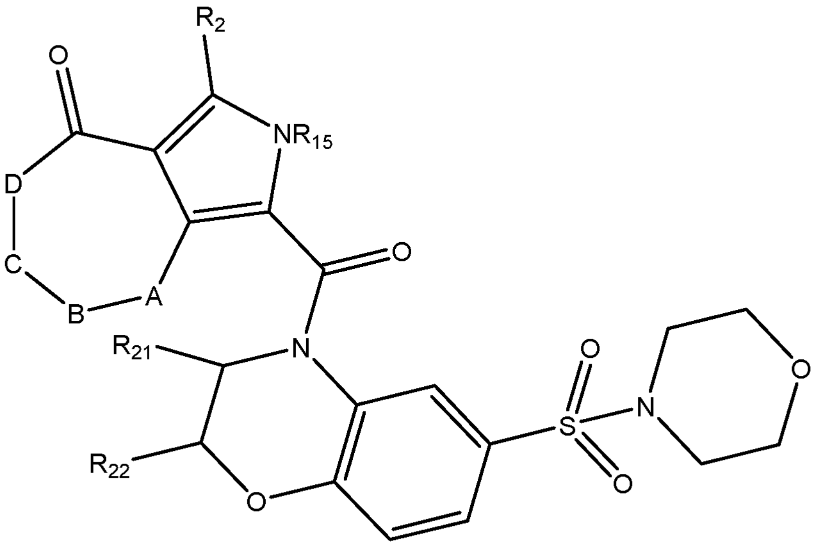

(4)

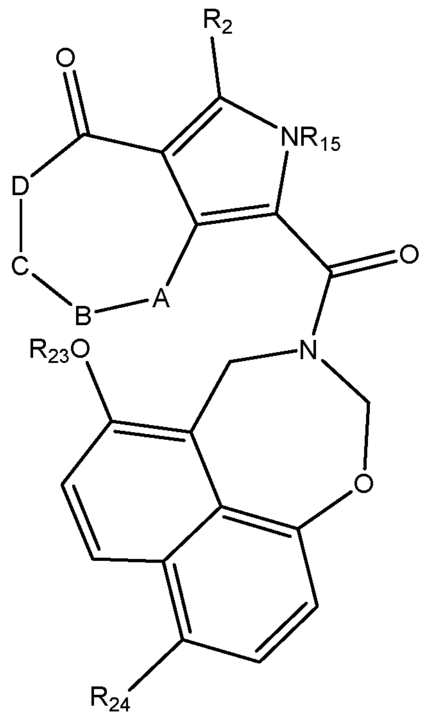

(5)

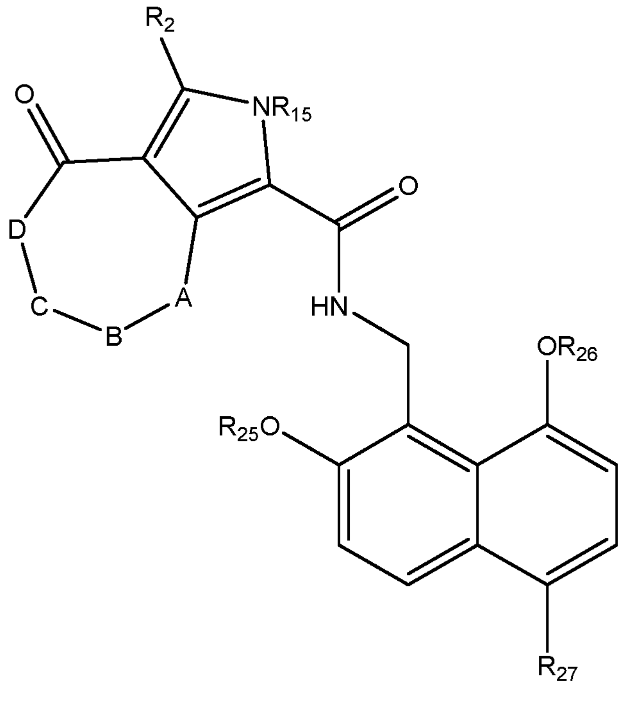

(6)

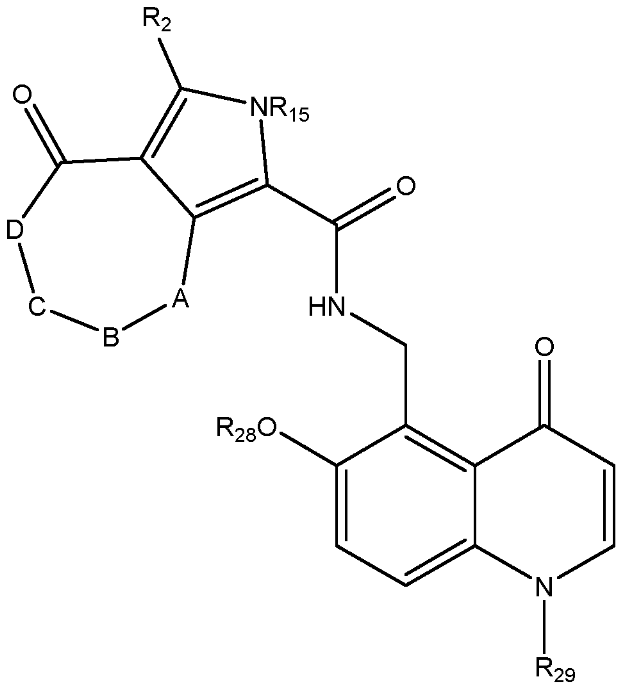

(7)

wobei A, B, C, D, $R_2$ und $R_{15}$ wie in Anspruch 1 definiert sind,
$R_{21}$ and $R_{22}$, unabhängig voneinander, Wasserstoff oder $C_1$-$C_4$-Alkyl sind oder zusammen eine chemische Bindung bilden,
$R_{23}$, $R_{25}$ und $R_{28}$, unabhängig voneinander, Wasserstoff oder $C_1$ to $C_4$-Alkyl sind,
$R_{24}$, $R_{27}$ and $R_{29}$, unabhängig voneinander, $C_1$-$C_4$-Alkyl oder $C_2$-$C_4$-Carboxyl sind, und
$R_{26}$ Wasserstoff oder $C_1$ bis $C_4$-Alkyl ist.

**13.** Verbindung der Formel (1) oder (2) gemäß einem der Ansprüche 1 bis 12 zur Verwendung in der Behandlung von Krankheiten oder Bedingungen, für die ein Bromodomain-Inhibitor indiziert ist.

**14.** Pharmazeutische Zusammensetzung enthaltend mindestens eine Verbindung der Formel (1) oder (2) wie in einem der Ansprüche 1 bis 12 definiert oder ein pharmazeutisch akzeptables Salz derselben und einen oder mehrere pharmazeutisch akzeptable Trägersubstanzen, Verdünnungsmittel oder Hilfsmittel.

**15.** Pharmazeutisches Kombinationsprodukt enthaltend mindestens eine Verbindung der Formel (1) oder (2) wie in einem der Ansprüche 1 bis 12 definiert oder ein pharmazeutisch akzeptables Salz derselben und einen oder mehrere pharmazeutisch akzeptable Trägersubstanzen, Verdünnungsmittel oder Hilfsmittel und einen oder mehrere weitere pharmazeutisch aktive Substanzen.

## Revendications

**1.** Composé de formule (1) ou de formula (2)

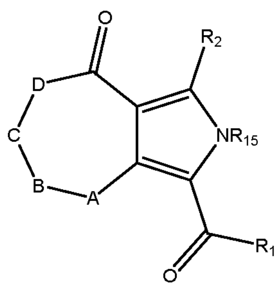

(1)

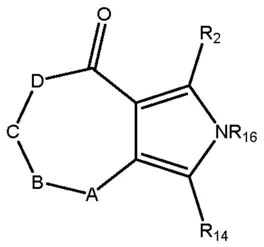

(2)

dans laquelle

A, B, C and D, qui peuvent être identiques ou différents, indépendants les uns des autres, représentent $-C(R_7R_8)-$, $-C(R_9R_{10})-C(R_{11}R_{12})-$ , $-N(R_{13})-$, -O-ou -S-, à condition que le nombre total d'atomes du cycle comprenant les éléments du cycle A, B, C et D soit de 7 à 9,
$R_1$ est choisi dans le groupe constitué de OH, $OR_3$, $NH_2$, $NHR_4$ et $NR_5R_6$ $R_2$ est un groupe alkyle substitué ou non substitué avec 1 à 8 atomes de carbone ou un groupe alcoxy substitué ou non substitué avec 1 à 8 atomes de carbone dans lequel un ou plusieurs des atomes d'hydrogène peuvent être éventuellement remplacés par un halogène,
$R_3$ and $R_4$, indépendants l'un de l'autre, représentent un groupe alkyle de 1 à 6 atomes de carbone ou un groupe alkylaryle, aryle ou hétéroaryle substitué ou non-substitué ,
$R_5$ and $R_6$, indépendants l'un de l'autre, représentent un groupe alkyle de 1 à 6 atomes de carbone ou un groupe aryle ou hétéroaryle substitué ou non-substitué ou forment avec l'atome d'azote auquel ils sont attachés un anneau hétérocyclique à six ou sept chaînons, substitué ou non-substitué, $R_7$ to $R_{13}$, indépendants les uns des autres, représentent l'hydrogène, l'hydroxyle, des groupes alkyle substitués ou non substitués avec 1 à 4 atomes de carbone, des groupes alcoxy substitués ou non substitués avec 1 à 4 atomes de carbone et des groupes hydroxyalkyle substitués ou non substitués avec 1 à 4 atomes de carbone ou dans lequel deux des $R_7$ à $R_{13}$ situés au niveau d'atomes adjacents forment ensemble un liaison chimique ou un anneau,
$R_{14}$ représente un groupe aryle ou hétéroaryle substitué ou non-substitué, , et
$R_{15}$ and $R_{16}$ indépendamment l'un de l'autre, sont un hydrogène ou un groupe alkyle en $C_1$ à $C_4$.

2. Composé de formule (1) ou (2) selon la revendication 1, dans lequel A, B, C et D sont -(CR7R8)-.

3. Composé de formule (1) ou (2) selon la revendication 1 ou 2, dans lequel $R_2$ est un groupe alkyle ou un groupe haloalkyle avec 1 à 4 atomes de carbone.

4. Composé de formule (1) selon l'une quelconque des revendications 1 à 3, dans lequel $R_1$ est un groupe NHR4 dans lequel $R_4$ est tel que défini dans la revendication 1.

5. Composé de formule (1) selon la revendication 4, dans lequel $R_4$ est un groupe aryle substitué ou non substitué, en particulier un groupe phényle substitué ou non substitué.

6. Composé de formule (1) selon la revendication 5, dans lequel $R_4$ est représenté par la formule (3)

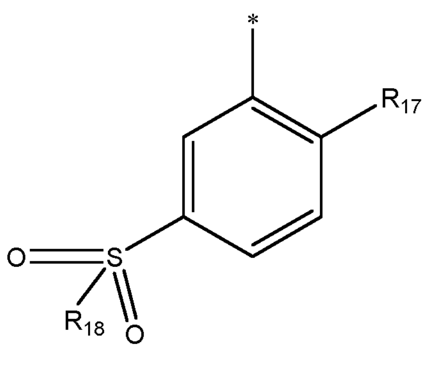

(3)

dans laquelle * représente le site de fixation à l'atome d'azote,

$R_{17}$ est un hydroxyle ou un groupe alcoxy avec 1 à 4 atomes de carbone et $R_{18}$ est $-NR_{19}R_{20}$ dans laquelle $R_{19}$ and $R_{20}$, indépendants l'un de l'autre, représentent un groupe alkyle avec 1 à 4 atomes de carbone, un cycle aryle substitué ou non substitué ou avec l'atome d'azote auquel ils sont attachés, ils forment un hétérocycle substitué ou non-substitué avec 4 à 7 atomes de cycle.

**7.** Composé de formule (1) selon l'une quelconque des revendications 1 à 3, dans lequel $R_1$ est un groupe -OR3, dans lequel $R_3$ est tel que défini dans la revendication 1.

**8.** Composé de formule (1) selon l'une quelconque des revendications 1 à 4 représenté par la formule (1")

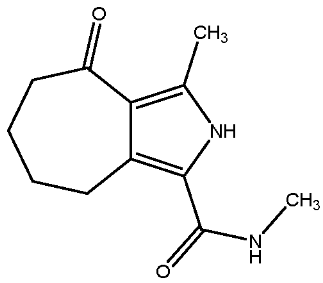

(1")

**9.** Composé de formule (2) selon l'une quelconque des revendications 1 à 3, dans lequel $R_{14}$ est un groupe phényle substitué ou non substitué ou un groupe oxadiazole substitué ou non substitué.

**10.** Composé de formule (2) selon l'une quelconque des revendications 1 à 3 ou la revendication 9, dans lequel $R_2$ est un groupe alkyle ayant 1 à 4 atomes de carbone.

**11.** Composé de formule (2) selon l'une quelconque des revendications 1 à 3, 9 ou 10, dans lequel $R_{14}$ est un groupe phényle ou oxadiazole substitué ou non-substitué.

**12.** Composé de formule (1) selon l'une quelconque des revendications 1 à 6 représenté par la formule (4), la formule (5), la formule (6) ou la formule (7)

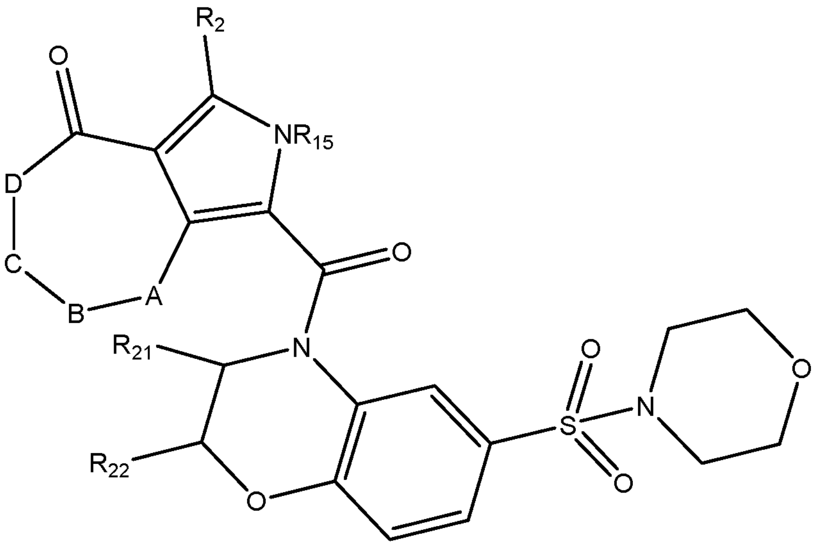

(4)

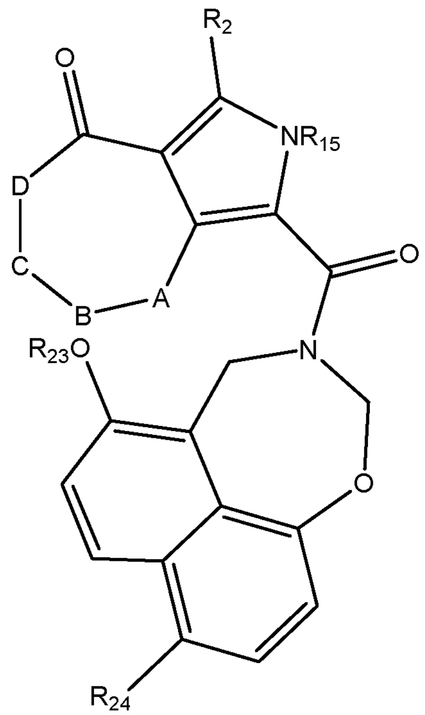

(5)

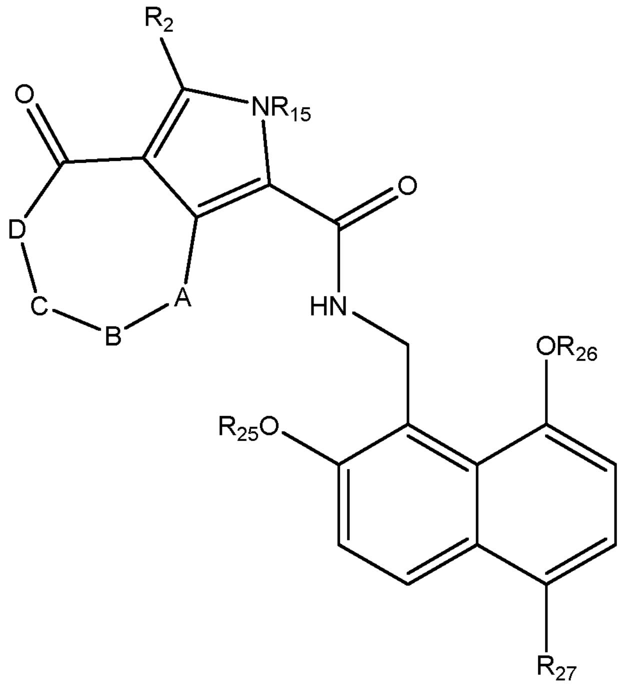

(6)

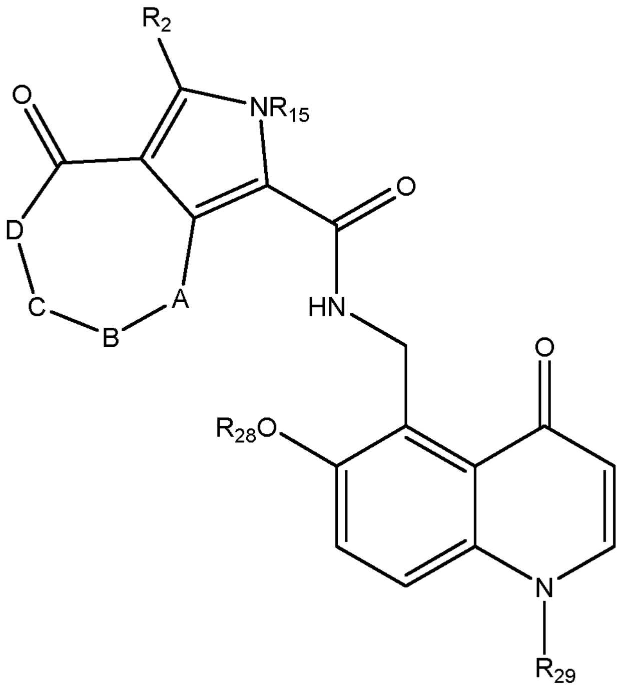

(7)

dans lequel A, B, C, D, $R_2$ et $R_{15}$ sont tels que définis dans la revendication 1,
$R_{21}$ and $R_{22}$, indépendants l'un de l'autre, représentent un hydrogène, un alkyle en $C_1$-$C_4$ ou forment ensemble une liaison chimique,
$R_{23}$, $R_{25}$ and $R_{28}$, indépendants l'un de l'autre, représentent un hydrogène ou un alkyle en $C_1$ à $C_4$,
$R_{24}$, $R_{27}$ and $R_{29}$, indépendants l'un de l'autre, représentent un alkyle en $C_1$-$C_4$ ou un carboxyle en $C_2$-$C_4$, et
$R_{26}$ représente un hydrogène ou un alkyle en $C_1$ à $C_4$.

**13.** Composé de formule (1) ou (2) selon l'une quelconque des revendications 1 à 11, destiné à être utilisé dans le traitement de maladies ou d'affections pour lesquelles un inhibiteur de bromodomaine est indiqué.

**14.** Composition pharmaceutique comprenant au moins un composé de formule (1) ou (2) tel que défini dans l'une quelconque des revendications 1 à 13 ou un sel pharmaceutiquement acceptable de celui-ci et un ou plusieurs véhicules, diluants ou excipients pharmaceutiquement acceptables.

**15.** Produit pharmaceutique combiné comprenant au moins un composé de formule (1) ou (2) tel que défini dans l'une quelconque des revendications 1 à 13 ou un sel pharmaceutiquement acceptable de celui-ci et un ou plusieurs véhicules, diluants ou excipients pharmaceutiquement acceptables conjointement avec un ou plusieurs autres ingrédients pharmaceutiquement actifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014164771 A
- US 2015111890 A
- US 20160200681 A
- WO 2014170350 A

### Non-patent literature cited in the description


- **FILIPPAKOPOULOS et al.** *Cell,* 2012, vol. 149 (1), 214-231
- **OWEN DJ et al.** The structural basis for the recognition of acetylated histone H4 by the bromodomain of histone acetyltransferase gcn5p. *The EMBO journal,* 2000, vol. 19 (22), 6141-6149
- **UMEHARA T et al.** Structural basis for acetylated histone H4 recognition by the human BRD2 bromodomain. *The Journal of biological chemistry,* 2010, vol. 285 (10), 7610-7618
- **MULLER S ; FILIPPAKOPOULOS P ; KNAPP S.** Bromodomains as therapeutic targets. *Expert reviews in molecular medicine,* 2011, vol. 13, e29
- **PRINJHA RK ; WITHERINGTON J ; LEE K.** Place your BETs: the therapeutic potential of bromodomains. *Trends in pharmacological sciences,* 2012, vol. 33 (3), 146-153
- **VIDLER LR ; BROWN N ; KNAPP S ; HOELDER S.** Druggability analysis and structural classification of bromodomain acetyl-lysine binding sites. *Journal of medicinal chemistry,* 2012, vol. 55 (17), 7346-7359
- **HASVOLD et al.** *Bioorganic & Medicinal Chemistry letters,* vol. 27 (10), 2225-2233
- **BERGE et al.** *J. Pharm.Sci.,* 1977, vol. 66, 1-19
- Burger's Medicinal Chemistry and drug discovery. Principles and Practice. vol. 1
- **FILIPPAKOPOULOS et al.** *Cell,* 2012, vol. 149 (1), 241-231
- **LESLIE AGW PH.** *Evolving Methods for Macromolecular Crystallography,* 2007, vol. 245 (41), 51
- **KABSCH W.** Xds. Acta crystallographica. Section D. *Biological crystallography,* 2010, vol. 66 (2), 125-132
- **BRUKER.** SADABS, SAINT and XPREP. Bruker AXS Inc, 2008
- The CCP4 suite: programs for protein crystallography. *Acta crystallographica, Section D, Biological crystallography,* 1994, vol. 50 (5), 760-763
- **EVANS P.** Scaling and assessment of data quality. *Acta crystallographica, Section D, Biological crystallography,* 2006, vol. 62 (1), 72-82
- **EVANS PR.** An introduction to data reduction: space-group determination, scaling and intensity statistics. *Acta crystallographica, Section D, Biological crystallography,* 2011, vol. 67 (4), 282-292
- **MCCOY AJ et al.** Phaser crystallographic software. *Journal of applied crystallography,* 2007, vol. 40 (4), 658-674
- **MURSHUDOV GN ; VAGIN AA ; DODSON EJ.** Refinement of macromolecular structures by the maximum-likelihood method. *Acta crystallographica. Section D, Biological crystallography,* 1997, vol. 53 (3), 240-255
- **MURSHUDOV GN et al.** REFMAC5 for the refinement of macromolecular crystal structures. *Acta crystallographica. Section D, Biological crystallography,* 2011, vol. 67 (4), 355-367
- **VAGUINE AA ; RICHELLE J ; WODAK SJ.** SFCHECK: a unified set of procedures for evaluating the quality of macromolecular structure-factor data and their agreement with the atomic model. *Acta crystallographica, Section D, Biological crystallography,* 1999, vol. 55 (1), 191-205
- **WLODEK S ; SKILLMAN AG ; NICHOLLS A.** Automated ligand placement and refinement with a combined force field and shape potential. *Acta crystallographica, Section D, Biological crystallography,* 2006, vol. 62 (7), 741-749